# EUROPEAN PATENT SPECIFICATION

(11) **EP 4 633 497 B1**
(45) Date of publication and mention of the grant of the patent: **18.11.2026**
(21) Application number: 23832887.6
(22) Date of filing: 20.11.2023
(51) Int. Cl.: A61B 17/70, A61B 17/80, A61B 17/04, A61B 17/88, A61B 90/90

(54) **MINIMALLY INVASIVE RIB FRACTURE ANCHORING FIXATION**
MINIMALINVASIVE RIPPENBRUCHVERANKERUNGSFIXIERUNG
FIXATION D'ANCRAGE D'UNE FRACTURE DE CÔTE MINIMALEMENT INVASIVE

(30) Priority: 12.12.2022 US 202263431823 P; 04.08.2023 US 202363530842 P
(43) Date of publication of application: 22.10.2025
(73) Proprietor: Zimmer Biomet CMF And Thoracic, LLC, Jacksonville, Florida 33218 (US)
(72) Inventor: MAXSON, William, Ponte Vedra, Florida 32081 (US); BONIFACE, Catherine, Jacksonville, Florida 32207 (US); FALENCKI, Thomas, Jacksonville, Florida 33218 (US); FEWELL, James, Neptune Beach, Florida 32266 (US); PETERS, Gary, Jacksonville, Florida 33218 (US); RAMOS, Jose, Jersey City, New Jersey 07302 (US); SUAREZ, Kerwin, Jacksonville, Florida 32256 (US); TOLBERT, Joshua, Jacksonville, Florida 33218 (US); WAHL, Joseph, Asbury Park, New Jersey 07712 (US); CASSONE, Guy, Jacksonville, Florida 33218 (US); SCHUBL, Sebastian, Jacksonville, Florida 33218 (US)
(74) Representative: Taor, Simon Edward William
(86) International application number: PCT/US2023/080567
(87) International publication number: WO 2024/129310

(56) References cited:
- WO-A1-2011/002882
- WO-A1-2014/150858
- WO-A1-2020/118087
- US-A1- 2019 142 485
- US-A1- 2019 290 258

## Description

### CLAIM OF PRIORITY

This application claims the benefit of U.S. Provisional Patent Application Serial No. 63/431,823, filed on December 12, 2022, and also claims the benefit of U.S. Provisional Patent Application Serial No. 63/530,842, filed on August 4, 2023, the benefit of priority of each of which is claimed hereby.

### BACKGROUND

Implants are common in the medical field for strengthening bones of patients. In some cases, implants can be attachable to bones that are broken. For example, when patients have cracked and broken (fractured) ribs, surgeons sometimes install a plate to maintain rib alignment to promote proper healing. A plate can be secured to the rib on either side of the fracture to maintain proper alignment of the rib during healing. The plate can be secured to the rib using fasteners. In some cases, the plate and fasteners can be removed from the patient after the fracture has healed.

An implant assembly with the features as defined in the preamble of claim 1 is known from WO 2014/150858 A1.

### SUMMARY OF THE INVENTION

The present invention is defined in claim 1 while preferred embodiments are set forth in the dependent claims.

Associated surgical methods are also described herein to aid understanding the invention. These methods do not form part of the claimed invention.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
FIG. 1 illustrates an isometric view of an implant assembly.
FIG. 2 illustrates a perspective view of an implant assembly.
FIG. 3 illustrates an isometric view of an implant assembly.
FIG. 4A illustrates a perspective view of an implant assembly.
FIG. 4B illustrates an enlarged perspective view of an implant assembly.
FIG. 5 illustrates an isometric view of an implant assembly.
FIG. 6 illustrates a perspective view of an implant assembly.
FIG. 7 illustrates an isometric exploded view of an implant assembly.
FIG. 8A illustrates cross-sectional view of an implant assembly.
FIG. 8B illustrates a schematic view of a portion of an implant assembly.
FIG. 8C illustrates a schematic view of a portion of an implant assembly.
FIG. 9A illustrates an isometric view of a portion of an implant assembly.
FIG. 9B illustrates an isometric view of a portion of an implant assembly.
FIG. 10A illustrates an isometric view of a portion of an implant assembly.
FIG. 10B illustrates an isometric view of a portion of an implant assembly.
FIG. 11A illustrates a perspective view of a portion of an implant assembly.
FIG. 11B illustrates a cross-sectional view of a portion of an implant assembly.
FIG. 12A illustrates a side view of a portion of an implant assembly.
FIG. 12B illustrates a cross-sectional view of a portion of an implant assembly.
FIG. 13A illustrates a perspective view of a portion of an implant assembly.
FIG. 13B illustrates a perspective view of a portion of an implant assembly.
FIG. 14A illustrates an isometric view of a portion of an implant assembly.
FIG. 14B illustrates an isometric view of a portion of an implant assembly.
FIG. 15A illustrates an isometric view of a portion of an implant assembly.
FIG. 15B illustrates an isometric view of a portion of an implant assembly.
FIG. 15C illustrates an isometric view of a portion of an implant assembly.
FIG. 16A illustrates an isometric view of a portion of an implant assembly.
FIG. 16B illustrates an isometric view of a portion of an implant assembly.
FIG. 16C illustrates an isometric view of a portion of an implant assembly.
FIG. 16D illustrates an isometric view of a portion of an implant assembly.
FIG. 16E illustrates an isometric view of a portion of an implant assembly.
FIG. 17 illustrates an isometric view of a portion of an implant assembly.
FIG. 18 illustrates an isometric view of a portion of an instrument assembly.
FIG. 19 illustrates an isometric view of a portion of an instrument assembly.
FIG. 20 illustrates an enlarged perspective view of an implant assembly.
FIG. 21A illustrates an isometric view of a portion of an implant assembly.
FIG. 21B illustrates an isometric view of a portion of an implant assembly.
FIG. 21C illustrates an isometric view of a portion of an implant assembly.
FIG. 22 illustrates a perspective view of an implant assembly.
FIG. 23 illustrates a perspective view of an implant assembly.
FIG. 24 illustrates a perspective view of an implant assembly.

### DETAILED DESCRIPTION

In some cases of fractured ribs where a plate is required to promote proper healing, fasteners can be used to secure the plate to the rib portions. For example, bone screws are often used to secure a plate to rib portions. However, use of screws alone to secure the plate to the rib portions can be relatively time consuming. Also, reduction of separated rib portions can be relatively difficult and time consuming and fasteners are not always useful for the reduction process.

The present application can include devices and methods to help address these issues, such as by including anchor assemblies which can include anchors (such as suture anchors) and brackets (e.g., buttons or fasteners) to secure the implant or plate to the bone and for more quickly reducing separated rib portions relative to traditional methods including screws and plates. The anchors can be operated intrathoracically or extrathoracically to quickly secure the implant to each rib portion following reduction, helping to reduce an amount of time required to complete a procedure.

In some cases of fractured ribs where a plate is required to promote proper healing, it may be desired to install the plate intrathoracically for reduced palpability and increased comfort. In these cases, it is common to install the plate from the opposite side of the thoracic cavity. For example, an anterior rib that is fractured at a ventral portion may be accessed from a posterior side of the patient. While this placement of an implant has many relative benefits, a procedure to place an implant as such can present several difficulties. For example, aligning the rib plate on the fractured rib and maintaining alignment for creation of bores in the rib and for fastening the plate to the rib can be a difficult process. The present application can include devices and methods to help address these issues, such as by using the anchor assemblies to relatively quickly reduce the rib portions and relatively quickly secure the implant to the bone.

The above discussion is intended to provide an overview of subject matter of the present patent application. It is not intended to provide an exclusive or exhaustive explanation of the invention. The description below is included to provide further information about the present patent application.

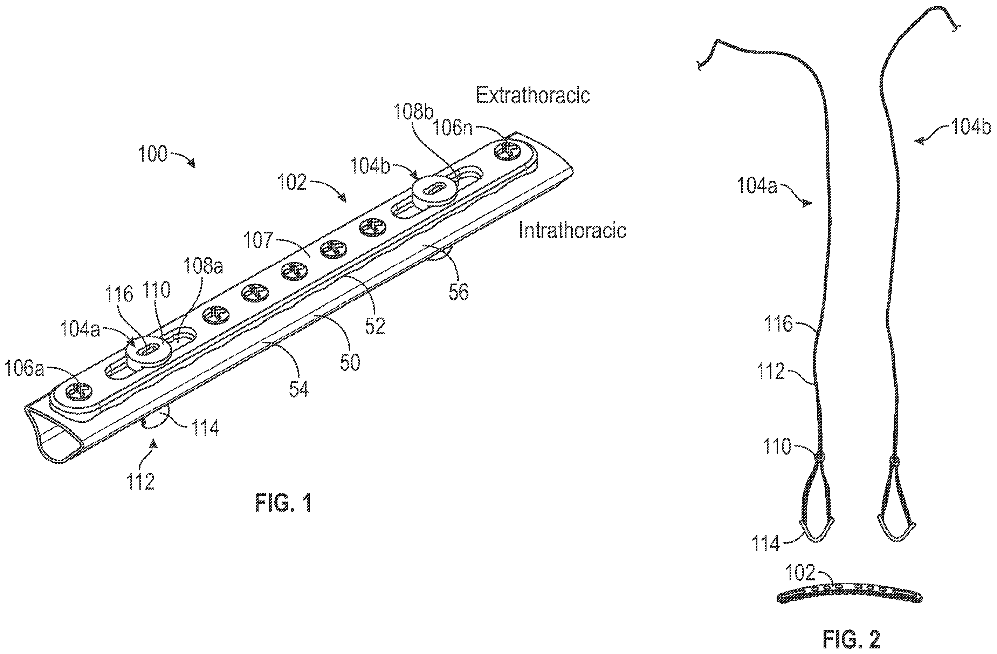

FIG. 1 illustrates an isometric view of an implant assembly 100 installed in a rib 50 using an extrathoracic approach. FIG. 2 illustrates a perspective view of the implant assembly 100. FIGS. 1 and 2 are discussed together below. FIG. 1 also shows a fracture 52 in the rib 50. The rib 50 can include a first rib portion 54 and a second rib portion 56. FIG. 1 also shows orientation indicators Extrathoracic and Intrathoracic.

The implant assembly 100 includes a plate 102, anchor assembly 104a and 104b, and fasteners 106a-106n. The plate 102 includes a body 107 and bores 108a and 108b. The body 107 can be curved such as to match an extrathoracic surface of the rib 50. The bores 108 can be round bores or can be elongated slots extending at least partially through the body 107. The bores 108a and 108b can be configured to receive the anchor assembly 104a and 104b, respectively at least partially therethrough. The body 107 can be elongate and can be a rigid or semi-rigid member. The body 107 can be made of biocompatible materials such as one or more of stainless steels, cobalt-chromium, titanium (e.g., commercially pure titanium (e.g., Grade 2) or titanium alloys), polymers such as polyether ether ketone (PEEK) or polyether ketone ketone (PEKK), or the like. Optionally, one or more components of the plate 102 or the anchor assemblies 104 could be made of one or more resorbable materials, such as Lactosorb, or other biodegradable polymers.

The anchor assembly 104a includes a bracket 110 and an anchor 112. The bracket 110 can be a button, fastener, or the like. The bracket 110 can be rigid or semi-rigid and can be configured to engage a portion of the plate 102 or bone. The anchor 112 can be a flexible or non-rigid (or semi-rigid) component or group of components operable to connect the anchor assemblies 104 to a bone (e.g., the rib 50) and a plate (e.g., the plate 102).

As shown in FIG. 2, the anchor 112 includes an anchor sleeve 114 and a suture 116. The brackets 106 and the bracket 110 can be made of biocompatible materials such as such as one or more of stainless steels, cobalt-chromium, titanium (e.g., commercially pure titanium or titanium alloys), polymer, such as polyether ether ketone (PEEK) or polyether ketone ketone (PEKK), or the like.

The anchor sleeve 114 or the suture 116 can be made of relatively soft and flexible materials such as one or more of cotton, silk, nylon, vicryl, or the like. The anchor sleeve 114 or the suture 116 can each optionally be formed of braided material. The anchor sleeve 114 can be a sleeve defining a longitudinal bore extending at least partially therethrough. The anchor sleeve 114 can be deformable from a first shape, as shown in FIG. 2, to a second shape, as shown in FIG. 1. The suture 116 can extend at least partly through the longitudinal bore of the anchor sleeve 114 to couple the sleeve 114 to the suture 116.

As shown in FIG. 1, the anchor 112 can be insertable at least partially into a hole formed in the first rib portion 54 and at least partially into the bore 108a. The hole can be formed using a punch or a drill. An underside of the bracket 110 can be engageable with the implant body 107 and can be configured to receive at least a portion of the suture 116 therethrough. The anchor 112 can be inserted through the bore 108 and the rib 50, such that the sleeve 114 can engage an underside of the rib 50. Once the anchor 112 is in place, the suture 116 can be operated or manipulated to tighten the anchor 112 around the bracket 110 and the suture 116 can be operated or manipulated (e.g., pulled) to deform the anchor sleeve 114 to secure the bracket 110 and the anchor 112 to the implant and the rib 50.

When the suture 116 is at least partially tightened, the suture 116 of the anchor assembly 104a and the anchor assembly 104b can be used to align and reduce the first rib portion 54 and the second rib portion 56. Following reduction, the suture 116 can be tensioned to secure the first rib portion 54 and the second rib portion 56 relative to the plate 102. Once the anchor 112 is tensioned, excess of the suture 116 can be trimmed. In this way, the anchor assemblies can be used to quickly secure the plate 102 to the 50.

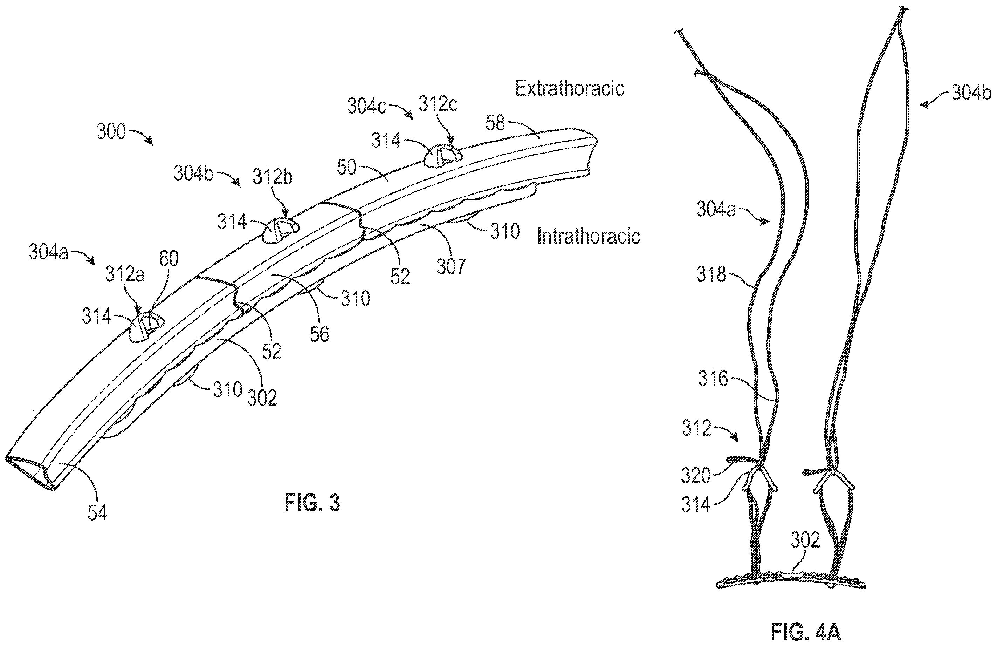

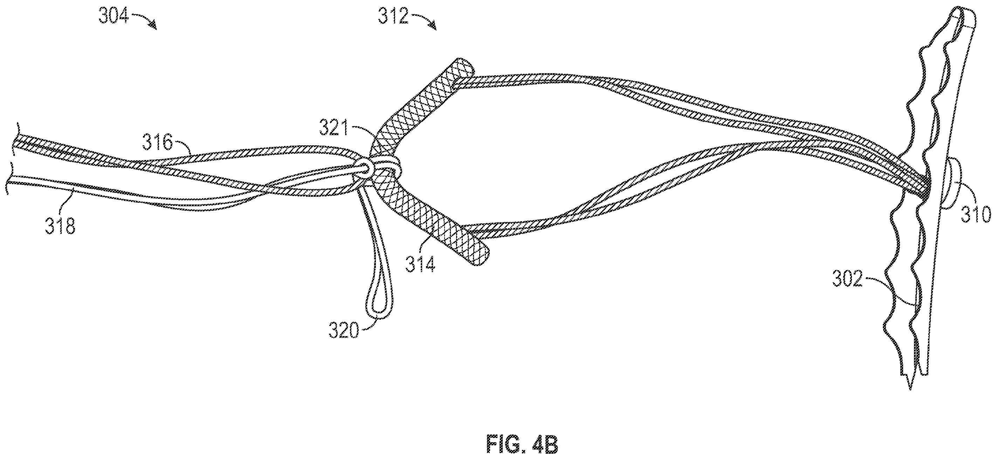

FIG. 3 illustrates an isometric view of an implant assembly 300 installed in a rib 50 using a hybrid intrathoracic or intrathoracic approach. FIG. 4A illustrates a perspective view of the implant assembly 300. FIG. 4B illustrates an enlarged view of the implant assembly 300. FIGS. 3-4B are discussed together below. FIG. 3 also shows a rib 50 including fractures 52. The rib can include a first rib portion 54, a second rib portion 56, and a third portion 58. FIG. 3 also shows orientation indicators Extrathoracic and Intrathoracic.

The implant assembly 300 can be similar to the implant assembly 100 discussed above, such that elements with similar reference numeral can be similarly configured. The implant assembly 300 can be configured for a hybrid intrathoracic and intrathoracic approach. Any of the implant assemblies discussed above or below can be modified to include the features of the implant assembly 300.

The implant assembly 300 can include a plate 302 including a body 307, which can be curved to match an intrathoracic surface of the rib 50. The body 307 can also include bores (which can be shaped as slots) configured to receive anchor assemblies 304a-304c therethrough. Each of the anchor assemblies 304 can include a bracket 310 and an anchor 312. The suture anchors 312 can each include an anchor sleeve 314, a suture 316, and a leader suture 318 including a tag 320. At least a portion of the suture 316 can extend through openings 321 (shown in FIG. 4B) of the anchor sleeve 314. The suture 316 can also extend at least partially through a longitudinal opening of the anchor sleeve 314 and can extend through the bracket 310. The leader suture 318 can be connected to the anchor sleeve 314.

In assembly, the plate 302 can be insertable intrathoracically to engage the rib 50. The anchor 312 can be insertable intrathoracically to extrathoracically at least partially into a bore (similar to the bore 108) of the plate 302 and at least partially into a hole 60 formed in the first rib portion 54. For example, the leader suture 318 can be inserted through the plate 302 and the hole 60 and can be used to pull the anchor sleeve 314 through the body 307 and the hole 60 without deforming the anchor sleeve 314. An underside of the bracket 310 can be engaged with the implant body 307.

When the anchor sleeve 314 is inserted through the bores of the plate 302 and the hole 60 of the rib 50 (e.g., the first rib portion 54), the sleeve 314 can engage an extrathoracic side of the rib 50. At this point, the tag 320 can be operated or manipulated to release the leader suture 318 from the anchor sleeve 314, allowing the leader suture 318 to be removed or disengaged from the anchor 312 (e.g., from the anchor sleeve 314).

This process can be repeated for each of the anchor assemblies 304. The suture 316 can then be operated or manipulated (such as extrathoracically) to pretension (beginning to tighten) the anchor 312 such as to cause the bracket 310 to engage the plate 302 and to cause the anchor sleeve 314 to at least partially deform (such as to compress or pre-compress, or change shape, such as shrinking in size) and engage the rib 50, causing the sleeve 314 and the bracket 310 to compress the plate 302 and the rib 50. When the anchor assemblies 304 are tightened, the anchor assemblies 304 can be used to align and reduce the rib portions 54, 56, and 58.

Once the rib portions 54, 56, and 58 are in place, the suture 316 can be further operated or manipulated to tighten the anchor 312 around the bracket 310 and the suture 316 can be operable to deform the anchor sleeve 314 to secure the bracket 310 and the anchor 312 to the implant and the rib 50. Each of the anchor assemblies 304 can be operated similarly such as to secure the anchor assemblies 304a, 304b, and 304c to the rib portions 54, 56, and 58, respectively, and to the plate 302. Once the anchors 312 are fully tightened, excess of the suture 316 can be trimmed. In this way, the anchor assemblies can be used to quickly secure the plate 302 to the rib 50.

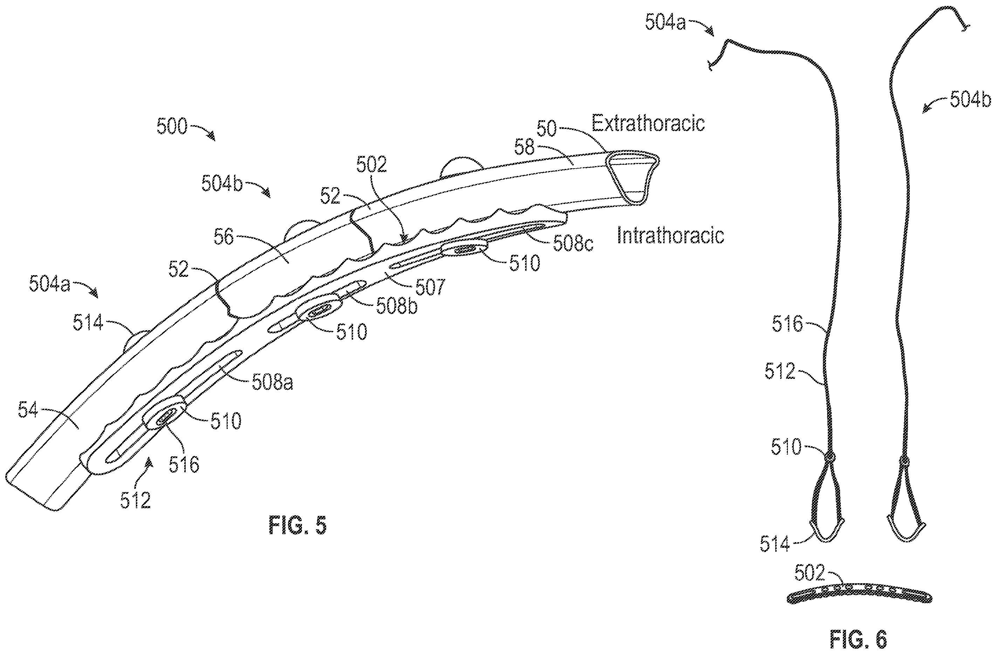

FIG. 5 illustrates an isometric view of an implant assembly 500 installed in a rib 50 using an intrathoracic approach. FIG. 6 illustrates a perspective view of the implant assembly 500. FIGS. 5 and 6 are discussed together below. FIG. 5 also shows a rib 50 including fractures 52. The rib can include a first rib portion 54, a second rib portion 56, and a third portion 58. FIG. 5 also shows orientation indicators Extrathoracic and Intrathoracic.

The implant assembly 500 can be similar to the implant assemblies discussed above, such that elements with similar reference numerals can be similarly configured. The implant assembly 500 can be configured for an intrathoracic approach. Any of the implant assemblies discussed above or below can be modified to include the features of the implant assembly 500.

More specifically, the implant assembly 500 can use the same components as the implant assembly 100 with a different plate, a plate 502. The plate 502 can also be rigid or semi-rigid and can be formed of biocompatible materials, but the plate 502 can be configured to engage an intrathoracic face of the rib 50 and therefor can have a curvature that is more similar to the plate 302. The plate 502 can include bores or slots 508a, 508b, and 508c, which can be configured to receive anchor assembles 504a, 504b, and 504c, respectively, at least partially therethrough.

In operation or installation of the implant assembly 500, an anchor 512 of an anchor assembly 504a can be inserted through a slot (e.g., the slot 508a) and a hole in the rib 50 to insert an anchor sleeve 514 and a suture 516 at least partially therethrough intrathoracically to extrathoracically. The suture 516 can be manipulated or operated (such as intrathoracically) to at least partially tighten the anchor 512 and can cause a bracket 510 to engage an intrathoracic face of a body 507 of the plate 502 and to secure the anchor assembles 504a to the first rib portion 54. This process can be repeated for anchor assemblies 504b and 504c to secure them, respectively, to rib portions 56 and 58. The sutures 516 can be manipulated to align and reduce the rib portions 54-58. Once the rib portions are aligned, as desired, the suture 516 of each of the anchor assemblies 504 can be tightened to secure the anchor 512 and the bracket 510 of each of the anchor assemblies 504 to the rib 50 and the plate 502 and can be operated to deform the sleeve 514. The sutures 516 can be optionally tensioned using a tensioner and the sutures 516 can be trimmed to remove excess material. The implant assembly 500 can allow for each of the steps discussed above to be performed intrathoracically.

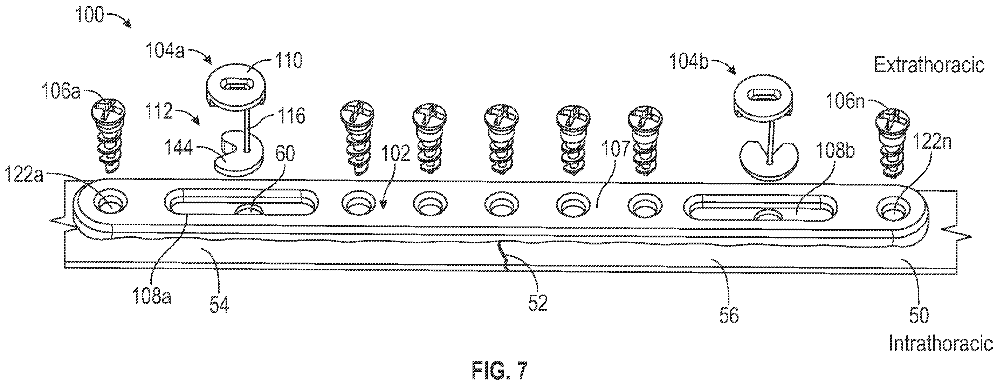

FIG. 7 illustrates an isometric exploded view of the implant assembly 100. FIG. 7 also shows a rib 50 including a fracture 52 and show rib portions 54 and 56. FIG. 7 also shows orientation indicators Extrathoracic and Intrathoracic.

The implant assembly 100 can be consistent with the implant assembly 100 discussed above. FIG. 7 shows additional details of the 100. For example, FIG. 7 shows that the body 107 can include a plurality of bores 122a-122n, where each can be configured to receive a fastener 106a-106n, respectively at least partially therein or therethrough, such as to help secure the plate 102 to the rib 50. Though 7 bores are shown, the plate 102 can include more or fewer bores, such as 1, 2, 3, 4, 5, 6, 8, 9, 10, 15, 20, or the like. Optionally, one or more of the bores 122 can be threaded to receive a fastener at least partially therein.

FIG. 7 also more clearly shows that the slots 108 can be elongate in shape (e.g., oval shape or stadium shape). FIG. 7 also shows that the bores 60 through the rib 50 can be located such that the plate 102 can be moved along a surface of the 50 (e.g., medially or laterally) with respect to the hole 60 and the bores 108 can still align with the hole 60, which can allow for the plate 102 to be positioned as required for proper reduction of the first rib portion 54 and the second rib portion 56 and proper securing of the plate 102 to the rib 50 following reduction.

FIG. 7 also shows the anchor assemblies 104 in a tightened configuration with excess suture 116 removed. In such a configuration, the anchor sleeve 114 can be deformed to a shape that prevents or limits the anchor sleeve 114 from moving through the hole 60. FIG. 7 also shows that the suture 116 can connect the bracket 110 to the anchor sleeve 114, such that, when the anchor assembly 104a is tightened, the anchor sleeve 114 can engage the rib 50 and the bracket 110 can engage the plate 102 to secure the anchor assemblies 104 and the plate 102 to the rib 50.

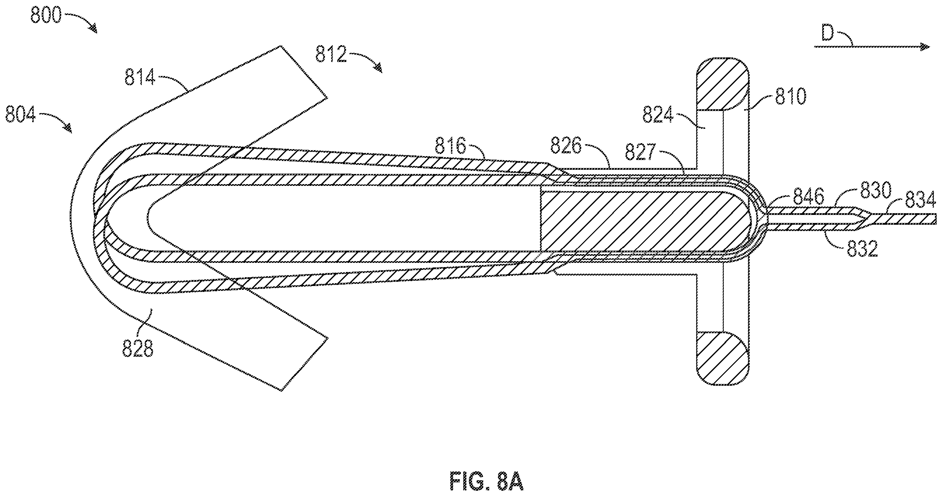

FIG. 8A illustrates cross-sectional view of an implant assembly 800. The implant assembly 800 can be similar to any of the implant assemblies discussed above. FIG. 8A shows how an anchor assembly can be connected in further detail. Any of the implant assemblies discussed above or below can be modified to include the features of the implant assembly 800.

The implant assembly 800 can include an anchor assembly 804 including a bracket 810 and an anchor 812. The anchor 812 can include an anchor sleeve 814 and a suture 816. The bracket 810 can include a head 824 and a body 826, which can together define a channel 827 extending at least partially through the bracket 810 and configured to retain at least a portion of the suture 816. The anchor sleeve 814 can include a lumen 828 through which the suture 816 can pass.

As discussed in further detail below, the suture 816 can include multiple parts or portions, but can be a single, continuous suture. The suture 816 can include a first free end 830 and a second free end 832 that can be independently manipulated or can be manipulated together. The first free end 830 and the second free end 832 can be connected to form a free end 834. The suture 816 can start at the first free end 830 and can pass into a bore or opening 846 and through the channel 827, and the suture 816 can extend through the lumen 828 to re-enter the channel 827 and can exit the channel 827 again to make another pass through the lumen 828 before the second free end 832 exits the bracket 810 and connects to the first free end 830.

The suture 816 can be connected and configured such that pulling on the free end 834 in the direction D (when the anchor sleeve 814 engages another surface, such as a rib), can cause the suture 816 to pull the bracket 810 in a direction opposite the direction D and can pull the anchor sleeve 814 in the direction D, effectively shortening the loops between the bracket 810 and the anchor sleeve 814. The pulling action can also cause the anchor sleeve 814 to deform as the suture 816 is shortened in length between the bracket 810 and the anchor sleeve 814, allowing the anchor assembly 804 to be used as an anchor.

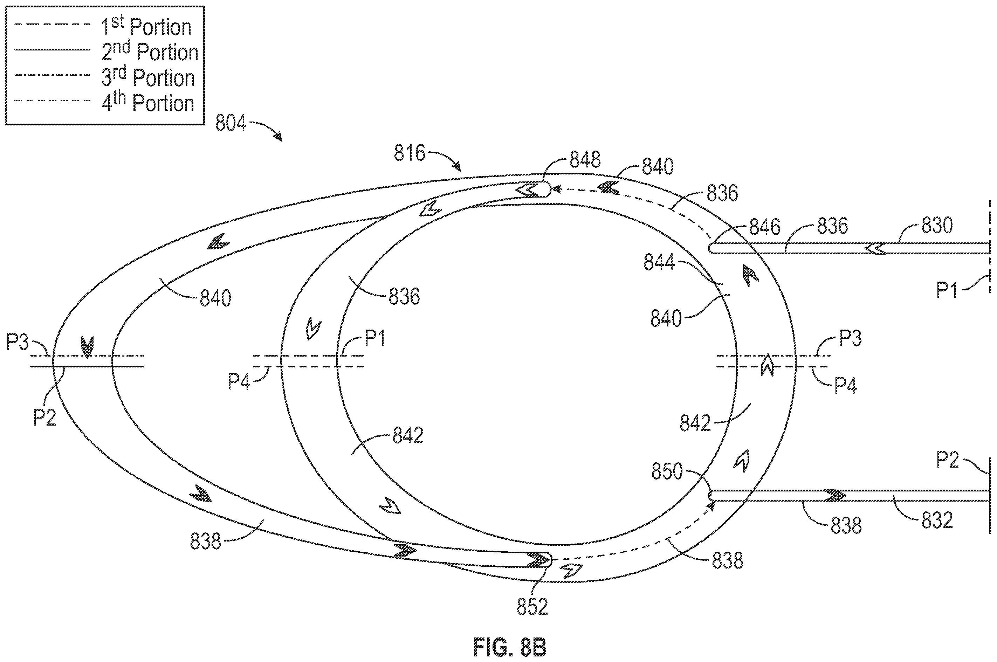

FIG. 8B illustrates a schematic view of a portion of an anchor assembly 804, which can be similar to any of the anchor assemblies discussed above. FIG. 8B shows how the suture 816 can be connected or arranged.

FIG. 8B illustrates the 816 schematically. The suture 816 can generally be a suture having a hollow core, allowing the 816 to pass through itself in certain places. The suture 816 includes a first portion 836 that includes the first free end 830. The suture 816 also includes a second portion 838 that includes the second free end 832. The first portion 836 can be a portion of the suture 816 between the lines P1-P1 and the second portion 838 can be a portion of the suture 816 between the lines P2-P2. The suture 816 also includes a third portion 840 that can be a portion of the suture 816 between the lines P3-P3 and a fourth portion 842 that can be a portion of the suture 816 between the lines P4-P4.

The suture 816 defines a lumen 844 extending therethrough, from the first free end 830 to the second free end 832. The third portion 840 can define a first end bore 846 and a first side bore 848. The fourth portion 842 defines a second end bore 850 and a second side bore 852. Optionally but not forming part of the invention, the bores 846 and 850 can be a single bore.

The suture 816 can be connected, as follows. The first portion 836 can extend into the first end bore 846 and into the third portion 840 and the lumen 844. The first portion 836 can extend out of the first side bore 848 and out of the lumen 844 and can transition into the fourth portion 842 at the lines P1 and P4. The first portion 836, the third portion 840, and the fourth portion 842 can together form a first loop. The fourth portion 842 can transition into the third portion 840 at the lines P3 and P4. The third portion can transition into the second portion at the lines P3 and P2. The fourth portion 842, the third portion 840, and the second portion 838 can together form a second loop. The second portion 838 can extend through the second side bore 852 and into the lumen 844 and the fourth portion 842. The second portion 838 can extend through the fourth portion 842 and through the second end bore 850 and out of the lumen 844.

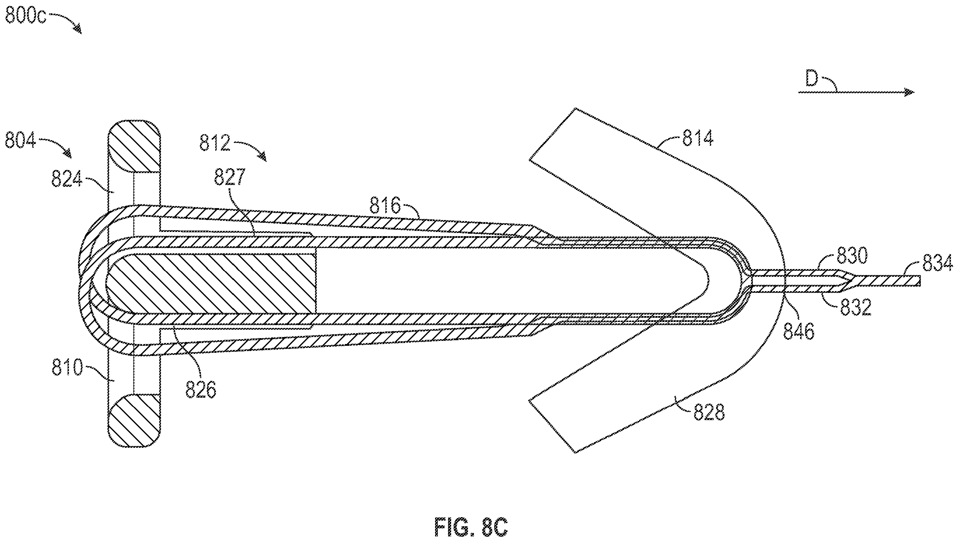

FIG. 8C illustrates cross-sectional view of an implant assembly 800C. The implant assembly 800C (which can include an anchor assembly 804 similar to the anchor assemblies 304 discussed above). FIG. 8C shows how an anchor assembly can be connected in further detail. Any of the implant assemblies discussed above or below can be modified to include the features of the implant assembly 800.

The implant assembly 800 includes an anchor assembly 804 including a bracket 810 and an anchor 812. The anchor 812 includes an anchor sleeve 814 and a suture 816. The bracket 810 can include a head 824 and a body 826, which can together define a channel 827 extending at least partially through the bracket 810 and configured to retain at least a portion of the suture 816. The anchor sleeve 814 includes a lumen 828 through which the suture 816 can pass.

As discussed in further detail below, the suture 816 includes multiple parts or portions, and is a single, continuous suture. The suture 816 includes a first free end 830 and a second free end 832 that can be independently manipulated or can be manipulated together. The first free end 830 and the second free end 832 can be connected to form a free end 834. The suture 816 can start at the first free end 830 and can enter a bore 846 and pass through the lumen 828 and into the channel 827. The suture 816 can wrap around the bracket 810 and can extend out of the channel 827 and can re-enter the 828, forming a loop. The suture 816 can pass through the channel 827 again and can enter the lumen 828 again to make another pass through the lumen 828, forming a second loop, before the second free end 832 exits the first end bore 846 and connects to the first free end 830.

The suture 816 can be connected and configured such that pulling on the free end 834 in the direction D (when the anchor sleeve 814 engages another surface, such as a rib), can cause the suture 816 to pull the sleeve 814 in a direction opposite the direction D and can pull the bracket 810 in the direction D, effectively shortening the loops between the bracket 810 and the anchor sleeve 814. The pulling action can also cause the anchor sleeve 814 to deform as the suture 816 is shortened in length between the bracket 810 and the anchor sleeve 814, allowing the anchor assembly 804 to be used as an anchor.

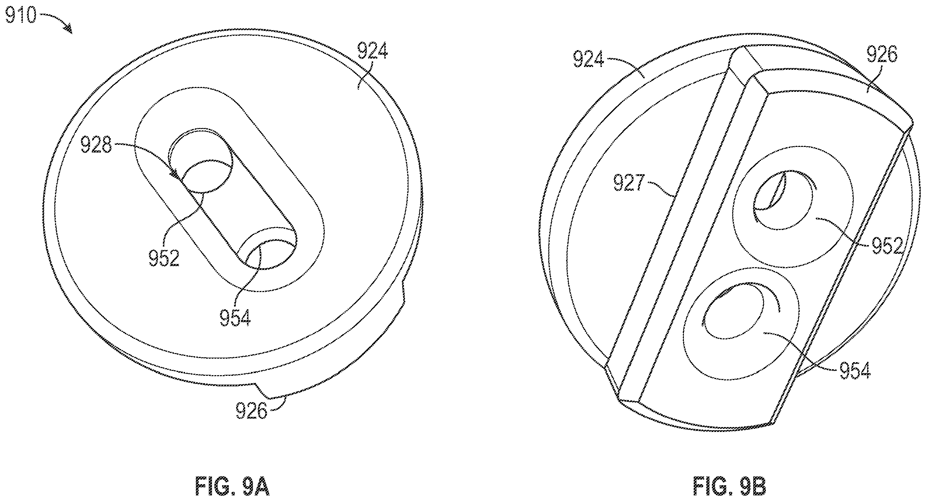

FIG. 9A illustrates an isometric view of a bracket 910 of an implant assembly. FIG. 9B illustrates an isometric view of the bracket 910 of an implant assembly. FIGS. 9A and 9B are discussed together below. The bracket 910 can be similar to the fasteners discussed above (e.g., the bracket 110). The bracket 910 can include a relatively smaller projection. Any of the implant assemblies discussed above or below can be modified to include the features of the bracket 910.

The bracket 910 can include a head 924 that can have a shape of a cylinder with a relatively small height and rounded edges. The bracket 910 can also include a projection 926 extending from one side of the head 924, such as from a base surface or top surface thereof. The projection can be configured (e.g., sized or shaped) to insert at least partially into one of the slots of any of the plates discussed above. The projection 926 can extend across a diameter (or a chord) of the head 924 and can define edges 927 that can be engageable with the slot of the plate. When inserted into the slot, the edges 927 of the projection 926 can engage the slot to limit relative rotation of the bracket 910 with respect to the slot and the plate.

The bracket 910 can also define a channel 928 configured to receive and retain a suture (e.g., the suture 116) at least partially within the bracket 910. The channel 928 can include a first bore 952 and a second bore 954 that can be configured to receive one or more loops of the suture (discussed above) therein. The bores 952 and 954 can be spaced from each other to retain a portion of the suture within the channel 928 and can allow for the bracket 910 to be drawn toward the plate and the anchor (e.g., the anchor sleeve 114) when the suture is operated to tighten the anchor assembly (e.g., the implant assembly 100). The channel 928 can also be at least partially recessed into the 924 such that extension of the suture above a top surface of the head 924 is limited.

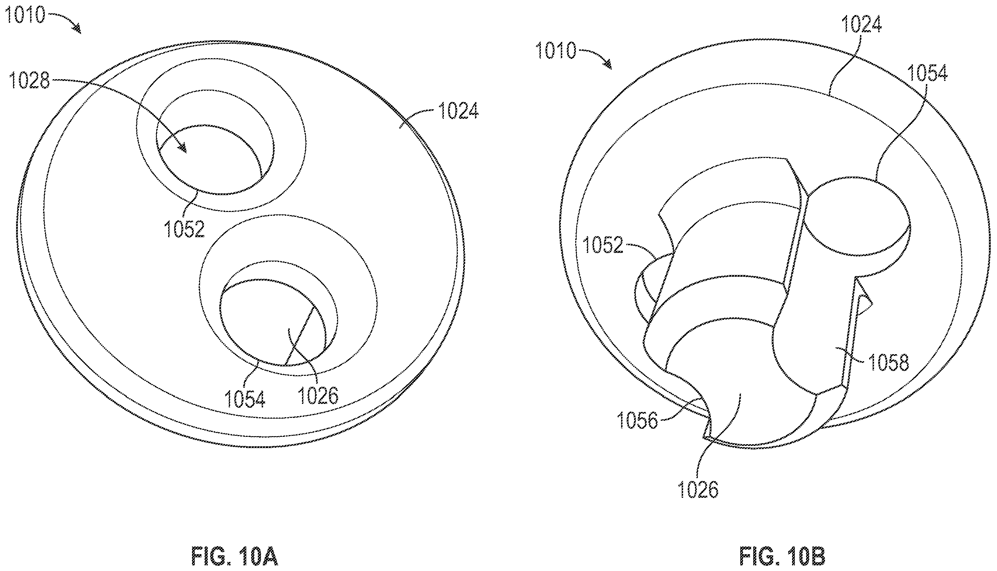

FIG. 10A illustrates an isometric view of a bracket 1010 of an implant assembly. FIG. 10B illustrates an isometric view of the bracket 1010 of an implant assembly. FIGS. 10A and 10B are discussed together below. The bracket 1010 can be similar to the fasteners (e.g., the bracket 110) discussed above. The bracket 1010 can include a relatively larger projection. Any of the implant assemblies discussed above or below can be modified to include the features of the bracket 1010.

The bracket 1010 can be similar to the bracket 910 discuss above, such that the bracket 1010 can include a head 1024, a projection 1026, and a channel 1028. The bracket 1010 can also define bores 1052 and 1054. The bracket 1010 can include the projection 1026 can be relatively cylindrical in shape with slots 1056 and 1058 extending along a length of the projection 1026. The projection 1026 can be inserted into the bore of the bone, which can help to limit movement of the rib with respect to the plate (e.g., the plate 102). The slots 1056 and 1058 can be configured to receive and retain at least a portion of a suture (e.g., the suture 116) therein when the suture 116 is connected to the bracket 1010.

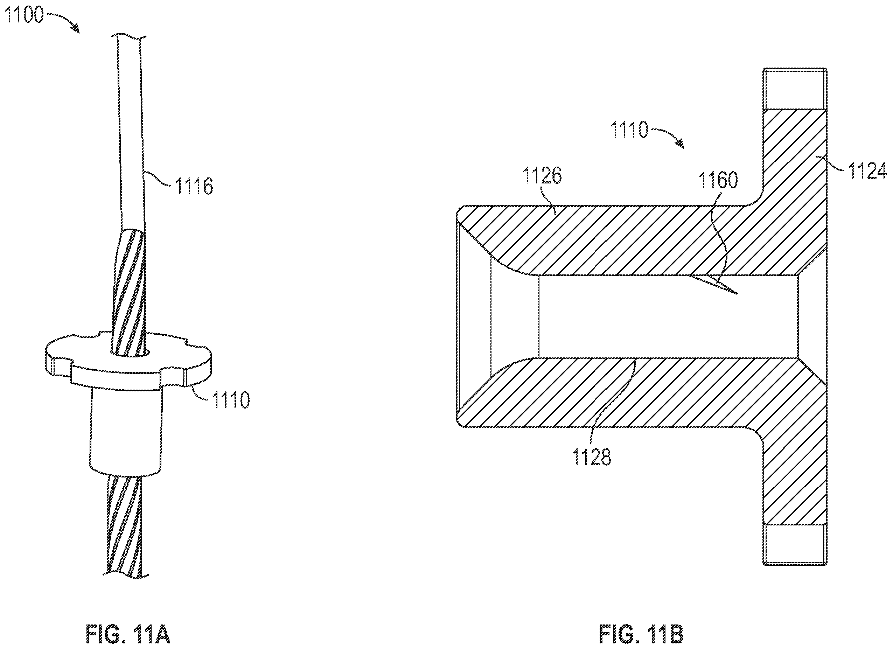

FIG. 11A illustrates a perspective view of a bracket 1110 and anchor 1116 of an implant assembly 1100. FIG. 11B illustrates cross-sectional view of the bracket 1110 of the implant assembly 1100. FIGS. 11A and 11B are discussed together below. The bracket 1110 can be similar to the fasteners discussed above (e.g., the bracket 110). The bracket 1110 can include a barb for limiting movement of the suture therethrough. Any of the implant assemblies discussed above or below can be modified to include the features of the bracket 1110. Optionally, the anchor 1116 can have vary in diameter along a length of the anchor 1116, such as to help reduce engagement between the anchor 1116 and the bracket 1110 until the bracket 1110 approaches the bone and plate (e.g., the plate 102).

The bracket 1110 can be similar to the fasteners discuss above, such that the bracket 1110 can include a head 1124, a projection 1126, and a channel 1128. The bracket 1110 can include the projection 1126 can include a barb 1160 (which can be a tooth or projection) configured to engage the anchor 1116 to limit movement of the anchor 1116 through the bracket 1110 to a single direction. That is, the bracket 1110 can be tightened onto the anchor 1116 but not loosened.

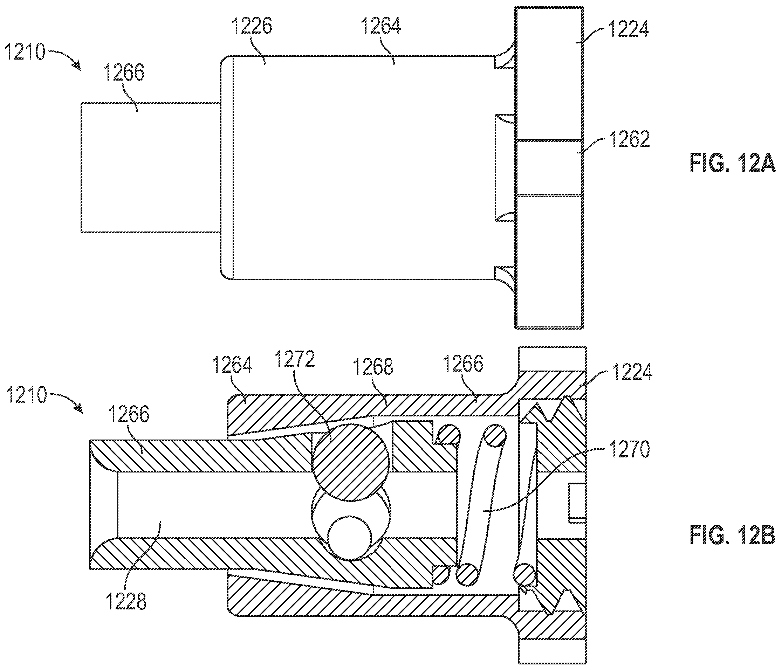

FIG. 12A illustrates a side view of a bracket 1210 of an implant assembly. FIG. 12B illustrates a cross-sectional view of the bracket 1210 of an implant assembly. FIGS. 12A and 12B are discussed together below. The bracket 1210 can be similar to the fasteners discussed above (e.g., the bracket 110). The bracket 1210 can include a locking assembly for limiting movement of the suture therethrough. Any of the implant assemblies discussed above or below can be modified to include the features of the bracket 1210.

The bracket 1210 can be similar to the fasteners discuss above, such that the bracket 1210 can include a head 1224, a projection 1226, and a channel 1228. The head 1224 can include notches 1262 that can be configured to receive an instrument therein, such as for removal or extraction of the bracket 1210.

The projection 1226 can include an outer body 1264 and an inner body 1266. The inner body 1266 can extend at least partially into a tapered bore 1268 of the outer body 1264. The inner body 1266 can also be tapered such that the inner body 1266 cannot extend entirely through the tapered bore 1268.

The bracket 1210 can also include a biasing element 1270, which can be a spring, such as a compression spring. The biasing element 1270 can be engaged with the head 1224 and the inner body 1266 to bias the inner body 1266 out of the tapered bore 1268. The head 1224 can also include movable features 1272, which can be, for example, balls or ball bearings. The balls 1272 can be located at least partially within the inner body 1266 and can be engageable with the tapered bore 1268 to extend into the channel 1228. In operation, the biasing element 1270 can bias the inner body 1266 to extend from the tapered bore 1268, causing the movable features 1272 to engage the tapered bore 1268 and extend into the channel 1228 such that the movable features 1272 can engage a suture (e.g., the suture 116) located within the 1228, limiting movement of the suture with respect to the bracket 1210.

Optionally, the inner body 1266 can be movable further into the tapered bore 1268 to overcome the biasing element 1270, allowing the movable features 1272 to move radially outward and disengage the suture within the channel 1228. In this way, the bracket 1210 can act as an automatically locking fastener that can be unlocked through movement of the inner body 1266 with respect to the outer body 1264.

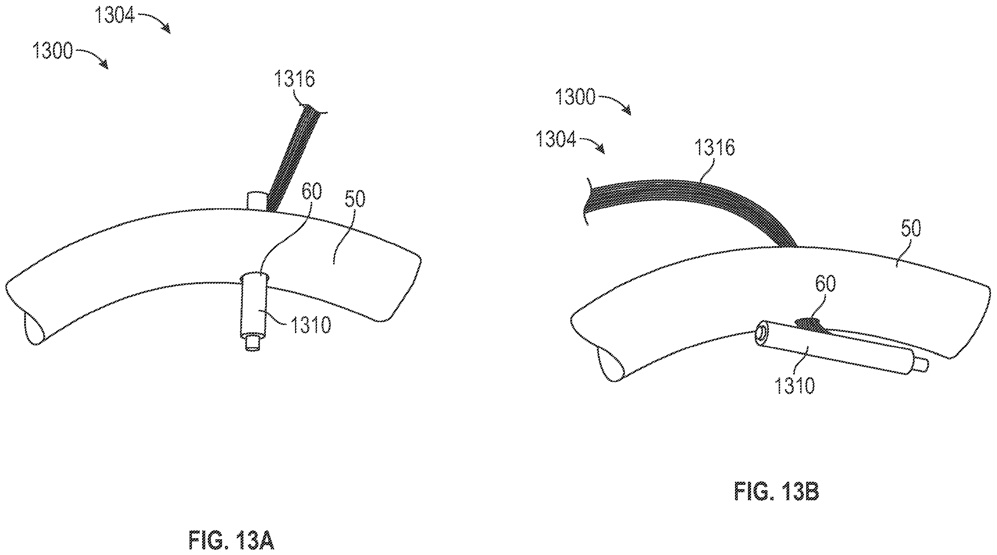

FIG. 13A illustrates a perspective view of a portion of an implant assembly 1300. FIG. 13B illustrates a perspective view of a portion of the implant assembly 1300. FIGS. 13A and 13B are discussed together below. The implant assembly 1300 can be similar to the implant assemblies discussed above (e.g., the implant assembly 100). The implant assembly 1300 can include a toggle anchor for securing an anchor assembly to the rib 50. Any of the implant assemblies discussed above or below can be modified to include the features of the implant assembly 1300.

More specifically, the implant assembly 1300 can include an anchor assembly 1304 including an anchor 1310 connected to a suture 1316. The anchor 1310 can be an elongate fastener having a rigid or semi-rigid body. For example, the anchor 1310 can have a shape of a cylinder or a hollow cylinder. The anchor 1310 can be sized or shaped such that when oriented to have its axis align with the hole 60, the anchor 1310 can be inserted to through a bore 60 of the rib 50, together with the 1316, as shown in FIG. 13A. Once the anchor 1310 is inserted entirely through the hole 60, the anchor 1310 can be rotated with or without assistance, such that its axis no longer aligns with the hole 60, as shown in FIG. 13B. Then, the anchor assembly 1304 can be tightened, pulling the anchor 1310 against a surface of the rib 50 and limiting the anchor 1310 from reinserting through the hole 60.

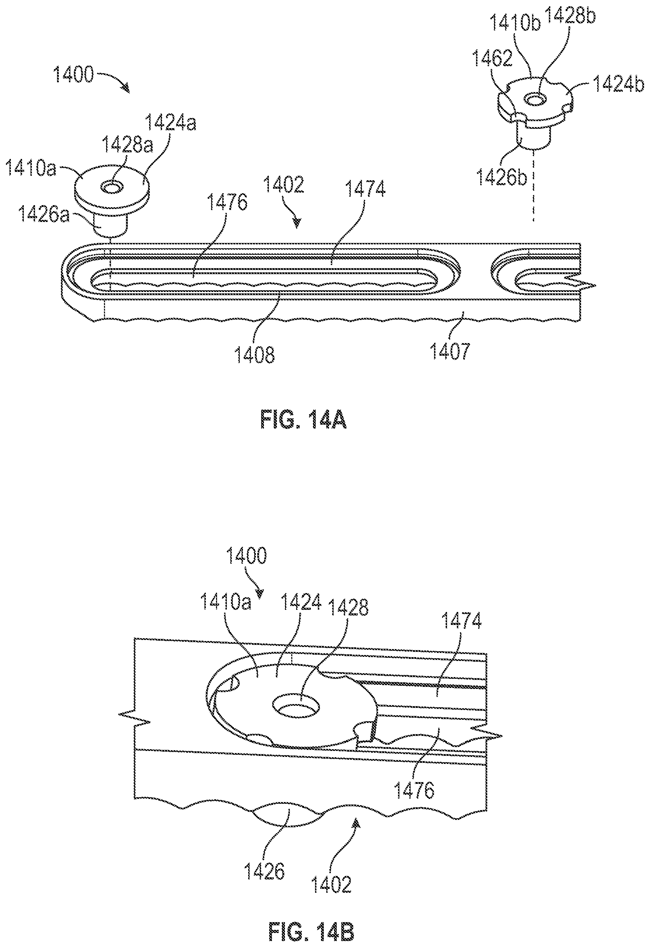

FIG. 14A illustrates an isometric view of a portion of an implant assembly 1400. FIG. 14B illustrates an isometric view of a portion of the implant assembly 1400. FIGS. 14A and 14B are discussed together below. The implant assembly 1400 can be similar to the implant assemblies discussed above (e.g., the implant assembly 100). Any of the implant assemblies discussed above or below can be modified to include the features of the implant assembly 1400.

FIG. 14A shows how the fasteners can have heads with different profiles. For example, a fastener 1410a can include a round head 1424a with a projection 1426a extending therefrom and a fastener 1410b can include a head 1424b including notches 1462. The notches 1462 can allow an instrument to engage the 1424 for adjustment, insertion, or extraction of the 1410b.

FIGS. 14A and 14B also show that a plate 1402 can include a body 1407 defining a slot 1408 therein. The slot 1408 can include a ledge 1474, such that the slot 1408 includes a counterbore defining the ledge 1474. The slot 1408 can also include a bore 1476 extending through the body 1407. The slot 1408 can be configured such that the projection 1426b can be insertable into and at least partially through the bore 1476 and such that extension or insertion of the brackets 1410 can be limited by engagement between the head 1424b and the ledge 1474. The ledge 1474 can be located or sized such that when the fastener 1410b is fully inserted into the 1408, the head 1424b does not extend beyond an outer surface of the body 1407.

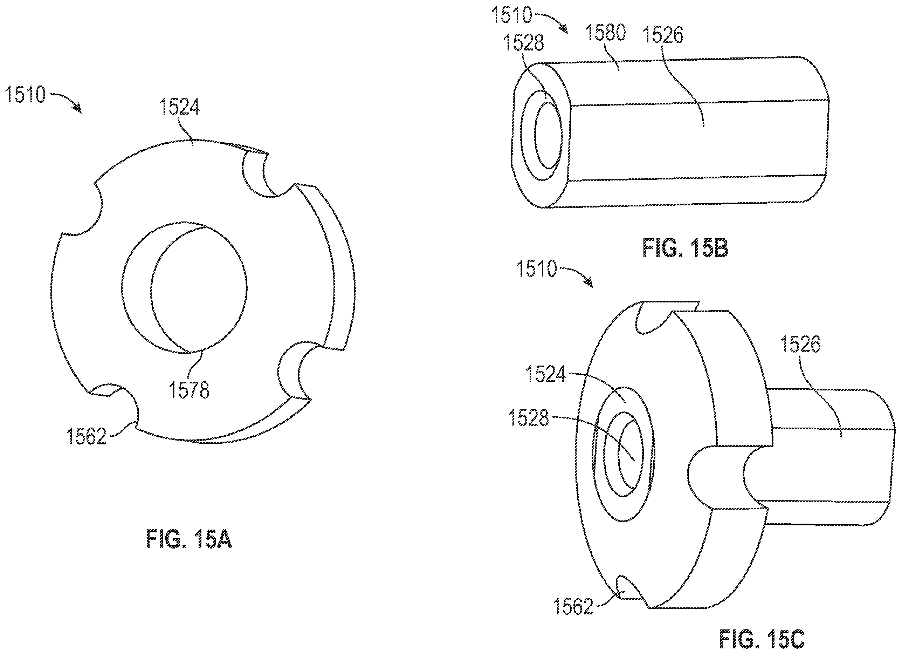

FIG. 15A illustrates an isometric view of a head of a bracket 1510 of an implant assembly. FIG. 15B illustrates an isometric view of a projection of the bracket 1510 of an implant assembly. FIG. 15C illustrates an isometric view of the bracket 1510 of an implant assembly. FIGS. 15A-15C are discussed together below.

The bracket 1510 can be similar to the fasteners discussed above, such as the bracket 110. The bracket 1510 can be an assembly of components. For example, the bracket 1510 can include a head 1524, as shown in FIG. 15A and a barrel 1526, as shown in FIG. 15B. The head 1524 can include notches 1562, which can be similar to the notches discussed above. The barrel 1526 can include a channel 1528 configured to receive at least a portion of an anchor therethrough.

The head 1524 can also include a threaded inner bore 1578 and the 1526 can include a threaded outer bore 1580 that can be threadably securable to the threaded inner bore 1578 to form the 1510, as shown in FIG. 15C. Through the use of the notches 1562, the head 1524 can be unthreaded from the barrel 1526 following assembly, such as for adjustment, revision, or extraction of the bracket 1510.

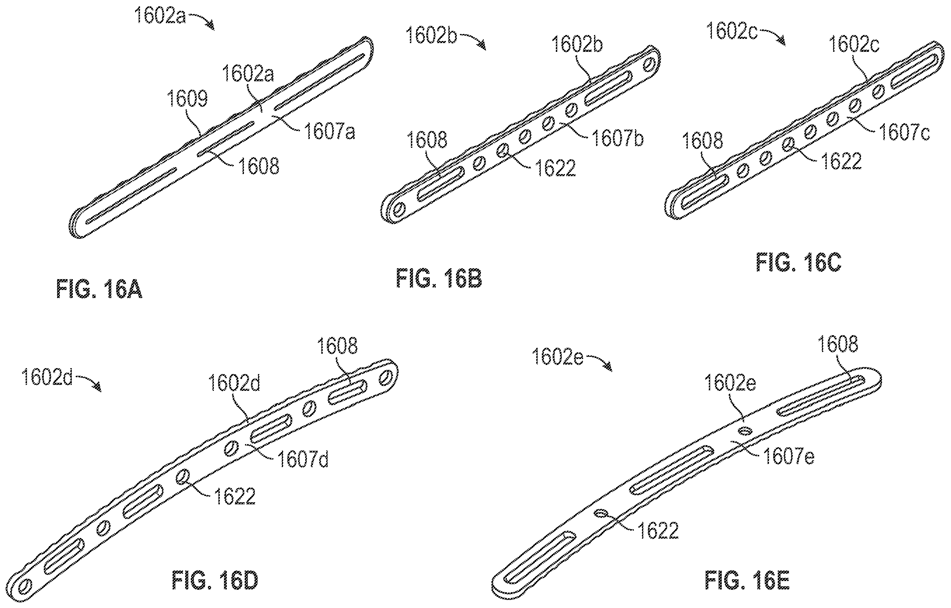

FIG. 16A illustrates an isometric view of a plate 1602A of an implant assembly. FIG. 16B illustrates an isometric view of a plate 1602B of an implant assembly. FIG. 16C illustrates an isometric view of a plate 1602C of an implant assembly. FIG. 16D illustrates an isometric view of a plate 1602D of an implant assembly. FIG. 16E illustrates an isometric view of a plate 1602E of an implant assembly. The plates 1602A-1602E are discussed together below.

Any of the plates 1602A-1602E can be incorporated into any of the implant assemblies discussed above. Any of the plates 1602 can be bendable, such as intraoperatively by a user (e.g. a surgeon) or during implantation (self-contourable) or can be provided pre-bent, to match a curvature of a rib of a patient. The plates 1602A-1602E can be of different shapes and sizes (e.g., length or width) with different features, such as for using in different procedures. Optionally, the plates 1602 can be malleable or bendable in various directions and along multiple axes to allow for patient-matched curvature of the plates 16023. For example, the plate 1602A can includes an elongate body 1607A defining slots 1608, such as three slots. The plate 1608A can be relatively flat and can be used in an intrathoracic approach procedure where fasteners are not used. Optionally, the plate 1602A can include one or more teeth, barbs, or projections 1609 configured to engage bone and limit movement of the plate 1602 with respect to the bone. Any of the plates discussed herein can include the projections 1609.

The plate 1602B can include a body 1607B defining multiple slots 1608, such as two slots and defining a plurality of fastener bores 1622, such as seven bores. The plate 1602B can include bores at lateral ends of the body 1607B to help secure the plate 1602B to a rib. Such a plate can be used in a procedure using an extrathoracic approach with one or more fractures.

The plate 1602C can include a body 1607C defining multiple slots 1608, such as two slots and defining a plurality of fastener bores 1622, such as seven bores. The plate 1602C can include all of the bores 1622 located between the slots 1608. Such a plate can be used in a procedure using an extrathoracic approach with one or more fractures.

The plate 1602D can include a body 1607D defining multiple slots 1608, such as four slots and defining a plurality of fastener bores 1622, such as six bores. The slots 1608 can be relatively evenly spaced with the fastener bores 1622, allowing the plate 1602D to be used in an extrathoracic approach with multiple fractures.

The plate 1602E can include a body 1607E defining multiple slots 1608, such as three slots and defining a plurality of fastener bores 1622, such as two bores. The slots 1608 can be relatively evenly spaced with the fastener bores 1622, allowing the plate 1602E to be used in an extrathoracic approach with multiple fractures.

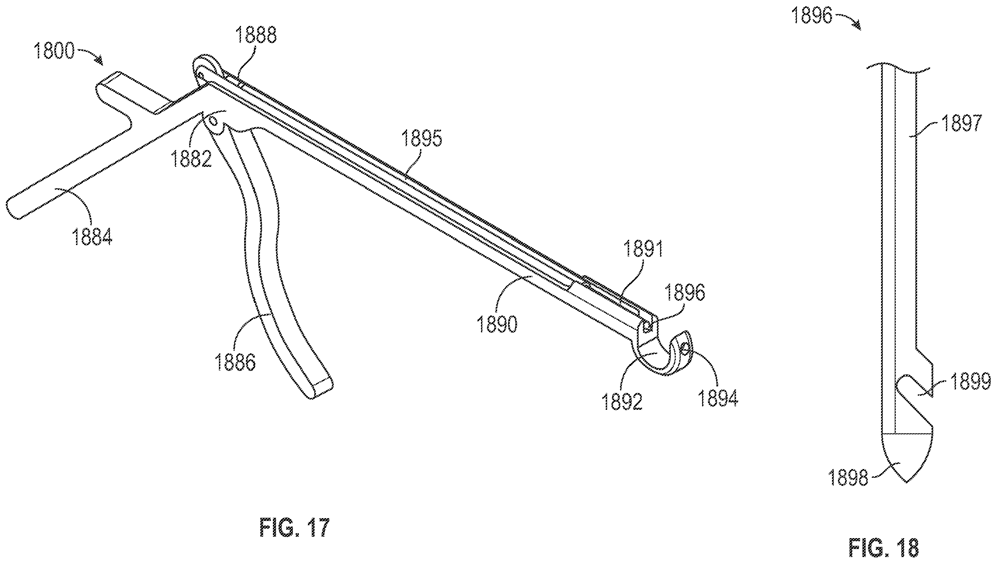

FIG. 17 illustrates an isometric view of an inserter 1800 (not claimed) of an implant assembly. FIG. 18 illustrates an isometric view of a portion of the inserter 1800. FIGS. 17 and 18 are discussed together below. The inserter 1800 can be used to form a hole in a bone, such as the hole 60 of the rib 50 and can be used to insert an implant assembly, such as the implant assembly 100 into the hole 60, as discussed in further detail below.

The inserter 1800 can include a body 1882 connected to a first handle 1884. The inserter 1800 can also include a second handle 1886 pivotably connected to the body 1882 and connected to an actuator 1888. The body 1882 can also include an arm 1890 defining a channel 1891. The arm 1890 can also include a retainer 1892 (or a guide) defining a bore 1894. The channel 1891 can be configured to receive and retain a punch 1896 (not claimed) at least partially therein. The inserter 1800 can also include a rod (or a shaft) 1895 engageable with or connectable to the punch 1896. Optionally, the punch 1896 can be coupled to the rod 1895 such that the punch 1896 can be disconnected from the rod 1895. Optionally, the rod 1895 and the punch 1896 can be connected through a threaded engagement or one or more fasteners.

The punch 1896 can include a shaft 1897 and a tip 1898 configured to punch through bone. The shaft 1897 or the tip 1898 can define a slot 1899 at least partially therein. The slot 1899 can be configured to receive and retain at least a portion of an anchor assembly (e.g., the anchor assembly 104) therein.

In operation, an anchor assembly can be connected to the punch 1896 and the punch 1896 can be inserted into the channel 1891. Then, the retainer 1892 can be hooked around a rib and optionally around a plate (e.g., the plate 102), such that the tip 1898 aligns with a channel (e.g., the bores 108) of the plate. The first handle 1884 and the second handle 1886 can then be operated to operate the actuator 1888, which can drive the punch 1896 out of the channel 1891 and into bone, forming a hole (e.g., the hole 60) in the bone (e.g., the rib 50) and passing at least a portion of the anchor assembly through the hole. The tip 1898 can optionally extend at least partially into the bore 1894.

Once the hole is formed, the punch 1896 can be retracted, either using the first handle 1884 and the second handle 1886, or by hand, and the punch 1896 can be removed from the newly formed hole. Optionally, the inserter 1800 can include a biasing member (such as a spring) engageable with the first handle 1884 and the second handle 1886 to bias the handles toward a retracted position, such as to automatically retract the second handle 1886 when the handles are released.

Due to the shape of the slot 1899, the anchor assembly can release from the punch 1896 when the punch 1896 is retracted, leaving at least a portion of the anchor assembly (e.g., the anchor sleeve 114) on an opposite side of the hole and leaving at least a portion of a suture (e.g., the suture 116) through the hole. Following formation of the hole and insertion of the anchor assembly, the process can be repeated as necessary, and the anchor assemblies (such as those discussed above) can be used to reduce rib portions and fix a plate to the rib, as discussed above.

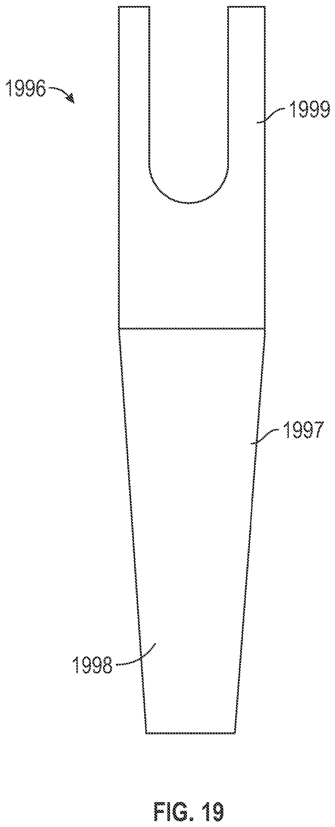

FIG. 19 illustrates an isometric view of a punch 1996 (not claimed) of an inserter. The
punch can be similar to the punch 1896 discussed above and can operate with the inserter 1800. The punch 1996 can include a shaft 1997 and a tip 1998 configured to punch through bone. The shaft 1997 or the tip 1998 can define a slot 1999 at least partially therein. The slot 1999 can be configured to receive and retain at least a portion of an anchor assembly (e.g., the anchor assembly 104) therein. Optionally, the shaft 1997 can be cannulated or can include a cannula such as for at least receiving at least a portion of an anchor assembly at least partially therein or therethrough.

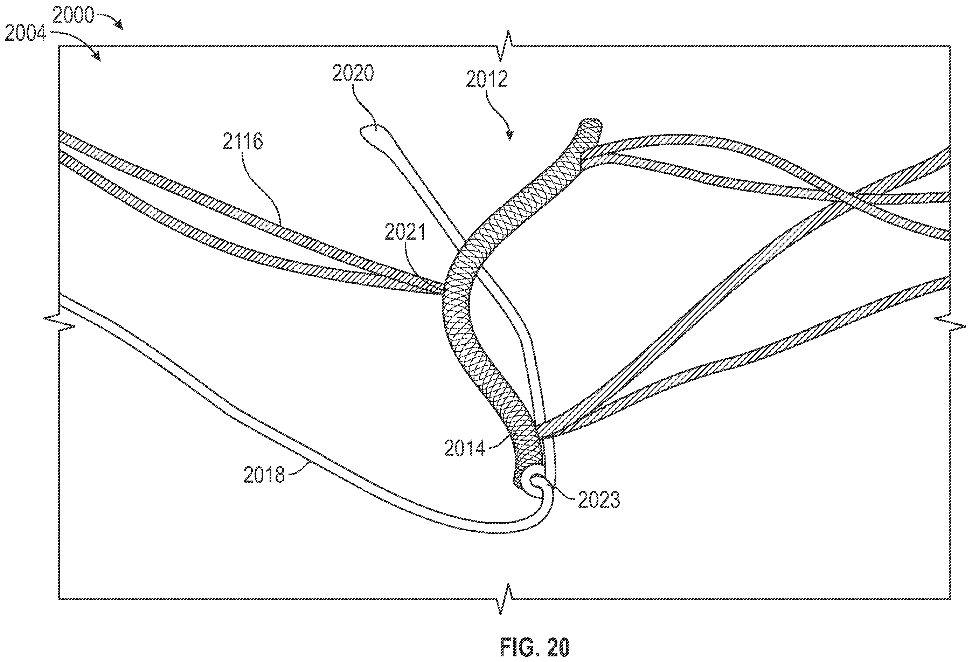

FIG. 20 illustrates an enlarged view of an implant assembly 2000, which can be similar to the implant assembly 300 discussed above, but can include a tag woven into an anchor sleeve. Any of the implant assemblies discussed above or below can be modified to include the features of the implant assembly 2000. Though only a portion of the implant assembly 2000 is shown, the implant assembly 2000 can include brackets, plates, and any of the other components of the implant assemblies discussed above or below.

The implant assembly 2000 can include an anchor assembly 2004, which can include a bracket and an anchor 2012. The anchor 2012 can include an anchor sleeve 2014, a suture 2016, and a leader suture 2018 including a tag 2020. At least a portion of the suture 2016 can extend through one or more openings of the anchor sleeve 314. The suture 2016 can also extend at least partially through a longitudinal opening of the anchor sleeve 2014 and can extend through a bracket. The leader suture 2018 can be connected to the anchor sleeve 2014, such as by being woven into the anchor sleeve 2014 at a connection portion 2023 of the leader suture 2018. Optionally, the leader suture 2018 can be interlaced with the anchor sleeve 2014.

In operation of some examples, the leader suture 2018 can be inserted through a plate and a hole of a rib and can be used to pull the anchor sleeve 2014 through the plate and the hole without deforming the anchor sleeve 2014. Because the leader suture 2018 is connected to an end (or near an end) of the anchor sleeve 2014, the anchor sleeve 2014 can be passed through the plate and the hole of the bone without folding in half, helping to reduce engagement and friction while passing the anchor 2012 through the plate and the bone, and helping to increase installation speed.

When the anchor sleeve 2014 is inserted through the bores of the plate and the hole of the rib (e.g., the first rib portion 54), the sleeve 2014 can engage an extrathoracic side of the rib 50. At this point, the tag 2020 can be operated or manipulated to release the leader suture 2018 from the anchor sleeve 2014, allowing the leader suture 2018 to be removed or disengaged from the anchor 2012 (e.g., from the anchor sleeve 2014).

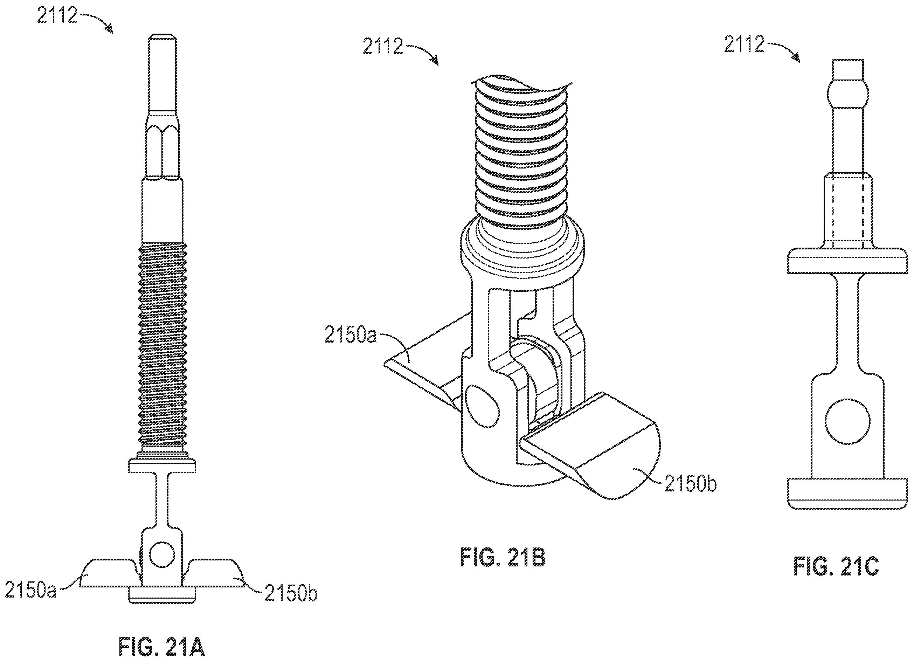

FIG. 21A illustrates an isometric view of an anchor 2112 of an implant assembly 2. FIG. 21B illustrates an isometric view of the anchor 2112 of an implant assembly. FIG. 21C illustrates an isometric view of the anchor 2112 of an implant assembly. The anchor 2112 can be similar to the anchors discussed above, the anchor 2112 can include deployable tabs. Any of the systems discussed above or below can be modified to include the features of the anchor 2112.

The anchor 2112 can include deployable tabs 2150 configured to deploy to extend radially outward following insertion of the anchor 2112 through a bone and plate. The tabs 2150 can engage the bone or plate following deployment, such as to help compress the bone and plate. The anchor 2112 can optionally include a threaded shank.

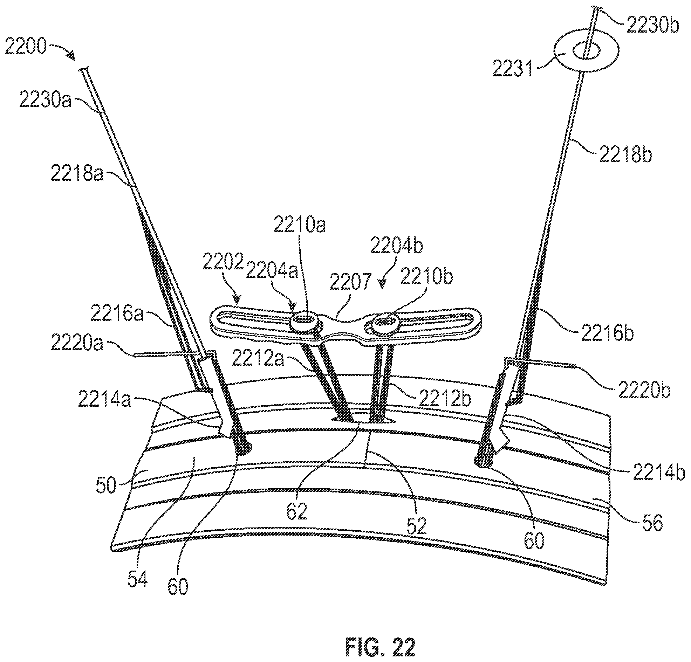

FIG. 22 illustrates a perspective view of an implant assembly 2200 installed in a rib 50 using a hybrid intrathoracic or intercostal approach for an intrathoracic plate. FIG. 22 also shows that the rib 50 includes fractures 52. The rib can include a first rib portion 54 and a second rib portion 56. FIG. 22 also shows an incision 62.

The implant assembly 2200 can be similar to the implant assemblies discussed above, such as the implant assembly 300 (or other assemblies), such that elements with similar reference numeral can be similarly configured. The implant assembly 2200 can be configured for a hybrid intrathoracic or intrathoracic approach to install an intrathoracic plate. Any of the implant assemblies discussed above or below can be modified to include the features of the implant assembly 2200.

The implant assembly 2200 can include a plate 2202 including a body 2207, which can be curved to match an intrathoracic or interior surface of the rib 50. The body 2207 can also include bores (which can be shaped as slots) configured to receive anchor assemblies 2204a and 2204b at least partially therethrough. Each of the anchor assemblies 2204a and 2204b can include a bracket 2210a and 2210b, respectively, and an anchor 2212a and 2212b, respectively. The anchors 2212 can be suture anchors and can each include an anchor sleeve 2214 (e.g., anchor sleeves 2214a and 2214b), and a suture 2216 (e.g., sutures 2216a and 2216b). At least a portion of the suture 2216 can extend through an opening (similar to the opening 321 shown in FIG. 4B) of the anchor sleeve 2214. The suture 2216 can also extend at least partially through a longitudinal opening of the anchor sleeve 2214 and can extend through the bracket 2210.

A leader suture 2218a can be connected to the anchor sleeve 2214a and a leader suture 2218b can be connected to the anchor sleeve 2214b. Optionally, a tag 2220 (e.g., 2220a and 2220b) can be connected to each of the leader suture 2218a and the leader suture 2218b to allow the tag to be manipulated or pulled to release the leader sutures 2218 from their respective anchor sleeves 2214. The tags 2220 can be similar to the tags 320 (or others discussed above). Optionally, the suture 2216a can be connected to the leader suture 2218a and the suture 2216b can be connected to the leader suture 2218b which can help to allow the sutures 2216 and the anchors sleeves 2214 to be pulled through the holes 60 without deforming the anchor sleeves 2214.

In operation or assembly, a first hole 60 can be formed in the first rib portion 54 and a second hole 60 can be in the rib portion 56, such as by drilling a hole through the first rib portion 54 and the rib portion 56. Also, the incision 62 can be created at a location or position near the rib 50, such as near the fracture 52. The leading suture 2218a and the suture 2216a can be inserted through the bracket 2210a (which can optionally be pre-assembled) and the implant or plate 2202 and the leading suture 2218b and the suture 2216b can be inserted through the bracket 2210b and the implant or plate 2202, such as until the anchors 2212 and anchor sleeves 2214 extend through the plate 2202. The leading sutures 2218a and 2218b and the sutures 2216a and 2216b can then be inserted into the incision 62 to enter a thoracic cavity of the patient and can be inserted through the holes 60 of the first rib portion 54 and the rib portions 56, respectively to exit the thoracic cavity. Optionally, multiple holes 60 can be created such that multiple anchor assemblies can be used within a single slot of the plate 2202.

Optionally, a snare can be inserted through the first hole into the thoracic cavity and out of the thoracic cavity through the incision 62 to grasp the free end 2230a with forceps (or a similar instrument) and the snare can be used to pull the free end 2230a through the incision and the hole 60 of the rib portion 54 and can be used to pull the free end 2230b through the incision and the hole 60 of the rib portion 56.

Once the leader sutures 2218 and the sutures 2216 are at least partially through the holes 60, the plate 2202 can be inserted through the incision 62 to engage an interior or intrathoracic portion of the first rib portion 54 and the rib portions 56, such as spanning the fractures 52. Also, the bracket 2210a and the bracket 2210b can be inserted through the incision to engage the plate 2202. After the plate 2202 is inserted through the incision 62 (or as the plate 2202 is being inserted), the plate 2202 can be rotated such that the curvature of the plate 2202 matches an internal or intrathoracic curvature of the rib 50.

The anchor sleeves 2214 of the anchor assembles 2204 can each be similar to the anchor sleeves discussed above (e.g., can be similar to the anchor sleeve 814 of the implant assembly 800C). The anchor sleeves 2214 can each define a longitudinal bore extending at least partially therethrough, such that the first anchor sleeve 2214a and the second anchor sleeve 2214b can each be deformable from a first shape to a second shape. And, the suture 2216a can extend at least partly through the longitudinal bore to couple the anchor sleeve 2214a to the suture 2216a. Similarly, the suture 2216b can extend at least partly through the longitudinal bore to couple the anchor sleeve 2214b to the suture 2216b.

When the plate 2202 and the anchor assemblies 2204a and 2204b are in place against the rib 50, the tags 2220 can be operated or pulled to release the leader sutures 2218 from their respective anchor sleeves 2214 prior to deformation of the anchor sleeves 2214. Then, the suture 2216a can be manipulated to tighten the first anchor assembly 2204a to the implant 2202 and the rib portion 54, and the suture 2216b can be manipulated to tighten the second anchor assembly 2204b to the implant 2202 and the rib portion 56. When the anchor assemblies are tightened, the free end 2230a can be manipulated to deform the anchor sleeve 2214a from a first shape to a second shape to tighten the anchor assembly 2204a to the implant 2202 and the rib portion 54. Similarly, the free end 2230b can be manipulated to deform the anchor sleeve 2214b from a first shape to a second shape to tighten the anchor assembly 2204b to the implant 2202 and the rib portion 56.

Either before deformation of the anchor sleeves, after deformation, or in partial deformation, the suture 2216a and the suture 2216b can be used to approximate or reduce the first rib portion 54 and the second rib portion 56. Following reduction, if necessary, the anchor sleeves 2214a and 2214b can be fully deformed to tighten the anchor assemblies 2204a and 2204b.

Optionally, prior to tightening or deformation, the suture 2216 of either anchor assembly can be inserted through a washer 2231. For example, the free end 2230b can be inserted through the washer 2231 and the anchor sleeve 2214b can base through the washer 2231 such that the washer 2231 engages the outer surface or extrathoracic portion of the rib portion 56. The anchor sleeve 2214b can then be tightened or deformed to engage the washer 2231 such that the washer 2231 is between the deformed anchor sleeve 2214b and the rib portion 56, which can help to reduce pressure on rib portion 56 near the hole 60, helping to limit pulling through of the anchor sleeve 2214b during or after tightening. Optionally, an open-sided washer can be placed around the suture 2216 such that the suture 2216 need not pass through the washer 2231.

Once the anchor sleeves 2214 are deformed, a tensioner can be applied to the first anchor assembly and the second anchor assembly to tension the anchor assemblies to a desired force or pressure. Optionally, the tensioner can be used to tension or tighten the first anchor assembly and the second anchor assembly multiple times. Following tensioning, the first suture 2216a and the second suture 2216b can be trimmed, such as near the first anchor sleeve 2214a and the second anchor sleeve 2214b, respectively.

Such a procedure can provide a minimally invasive process for repairing a fractured rib while placing an intrathoracic plate without deflating or "dropping" a lung for access.

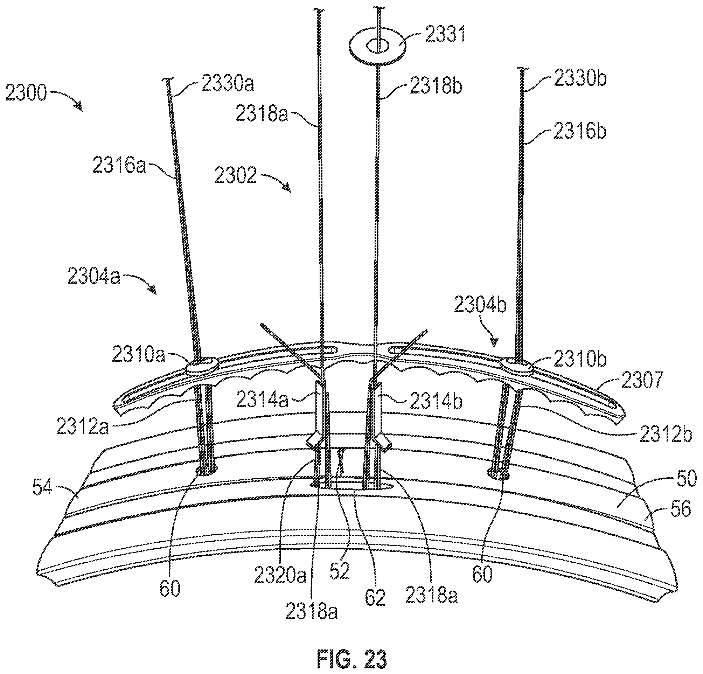

FIG. 23 illustrates a perspective view of an implant assembly 2300 installed in a rib 50 using an intercostal approach to installing an extrathoracic plate or implant. FIG. 23 also shows that the rib 50 includes fractures 52. The rib can include a first rib portion 54 and a second rib portion 56, and a third portion 58.

The implant assembly 2300 can be similar to the implant assemblies discussed above, such as the implant assembly 100 or 500 (or other assemblies), such that elements with similar reference numeral can be similarly configured; the implant assembly 2300 can include a leader suture 2318 attached to each anchor sleeve. The implant assembly 2300 can be configured for a intercostal approach for an extrathoracic plate. Any of the implant assemblies discussed above or below can be modified to include the features of the implant assembly 2300.

The implant assembly 2300 can include a plate 2302 including a body 2307, which can be curved to match an extrathoracic or exterior surface of the rib 50. The body 2307 can also include bores (which can be shaped as slots or elongate bores) configured to receive anchor assemblies 2304a and 2304b therethrough. Each of the anchor assemblies 2304a and 2304b can include a bracket 2310a and 2310b, respectively, and an anchor 2312a and 2312b, respectively. The anchors 2312 can be suture anchors and can each include an anchor sleeve 2314 (e.g., anchor sleeves 2314a and 2314b, and a suture 2316 (e.g., sutures 2316a and 2316b). At least a portion of the suture 2316 can extend through an opening of the bracket 2310. Each suture 2316 can also extend at least partially through a longitudinal opening of its respective anchor sleeve 2314. A leader suture 2318a can be connected to the anchor sleeve 2314a and a leader suture 2318b can be connected to the anchor sleeve 2314b. Optionally, a tag 2320 (e.g., 2320a) can be connected to each of the leader suture 2318a and the leader suture 2318b to allow the tag to be manipulated or pulled to release the leader sutures 2318 from their respective anchor sleeves 2314. The tags 2320 can be similar to the tags 320 (or others discussed above).

In operation or assembly, a first hole 60 can be formed in the first rib portion 54 and a second hole 60 can be in the rib portion 56, such as by drilling a hole through the first rib portion 54 and the rib portion 56. Also, the incision 62 can be created at a location or position near the rib 50, such as near the fracture 52. The leader suture 2318a can be inserted through the bracket 2310a and the implant or plate 2302 and the leader suture 2318b can be inserted through the bracket 2210b and the implant or plate 2302, such as until the anchors 2312 and anchor sleeves 2314 extend through the plate 2302 (which can optionally be pre-assembled).

The leader sutures 2318a and 2318b can then be inserted through the holes 60 of the first rib portion 54 and the rib portions 56, respectively, to enter the thoracic cavity, and can then be passed through the incision 62 to exit a thoracic cavity. Optionally, a snare or forceps can be inserted through the incision 62 to grasp the leader sutures 2318 to pull them through the incision 62.

Once the leader sutures 2318 are at least partially through the incision 62 (or prior thereto) (and optionally when the suture sleeves are through the incision 62), the plate 2302 can be inserted through an incision (e.g., an incision in addition to the incision 62 that can provide access to the rib 50) to engage an exterior or extrathoracic portion of the first rib portion 54 and the rib portions 56, such as spanning the fracture 52. Also, the bracket 2310a and the bracket 2310b can be inserted through the incision to engage the plate 2302 and can be optionally at least partially inserted into the holes 60. Optionally, the brackets 2310 can be pre-assembled with the plate 2302. At this point, the anchor sleeves 2314 can be moved to or located on an extrathoracic side of the rib 50.

The anchor sleeves 2314 of the anchor assembles 2304 can each be similar to the anchor sleeves discussed above (e.g., can be similar to the anchor sleeve 814 of the implant assembly 800). The anchor sleeves 2314 can each define a longitudinal bore extending at least partially therethrough, such that the first anchor sleeve 2314a and the second anchor sleeve 2314b can each be deformable from a first shape to a second shape. And, the suture 2316a can extend at least partly through the longitudinal bore to couple the anchor sleeve 2314a to the suture 2316a. Similarly, the suture 2316b can extend at least partly through the longitudinal bore to couple the anchor sleeve 2314b to the suture 2316b. The tags 2320 can be operated or pulled to release the leader sutures 2318 from their respective anchor sleeves 2314 prior to deformation of the anchor sleeves 2314.

When the plate 2302 and the anchor assemblies 2304a and 2304b are in place against the plate and the rib 50, the suture 2316a can be manipulated to tighten the first anchor assembly 2304a to the implant 2302 and the rib portion 54, and the suture 2316b can be manipulated to tighten the second anchor assembly 2304b to the implant 2202 and the rib portion 56. When the anchor assemblies are tightened, the free end 2330a can be manipulated to deform the anchor sleeve 2314a from a first shape to a second shape to tighten the anchor assembly 2304a to the implant 2302 and the rib portion 54. Similarly, the free end 2330b can be manipulated to deform the anchor sleeve 2314b from a first shape to a second shape to tighten the anchor assembly 2304b to the implant 2302 and the rib portion 56.

Either before deformation of the anchor sleeves, after deformation, or in partial deformation, the suture 2316a and the suture 2316b can be used to approximate or reduce the first rib portion 54 and the second rib portion 56. Following reduction, if necessary, the anchor sleeves 2314a and 2314b can be fully deformed to tighten the anchor assemblies 2304a and 2304b.

Optionally, prior to tightening or deformation and after removing the leading sutures 2320, the leader suture 2318 of either anchor assembly can be inserted through a washer 2331. For example, the leader suture 2318b can be inserted through the washer 2331 and the anchor sleeve 2314a can pass through the washer 2331 such that the washer 2331 engages the inner surface or intrathoracic portion of the rib portion 54. Optionally, the washer 2331 can be passed over the anchor sleeve 2314b following removal of the leader suture 2318b. The anchor sleeve 2314b can then be tightened or deformed to engage the washer 2331 such that the washer 2331 is between the deformed anchor sleeve 2314a and the rib portion 54, which can help to reduce pressure on rib portion 54 near the hole 60, helping to limit pulling through of the anchor sleeve 2314b during or after tightening.

Once the anchor sleeves 2314 are deformed, a tensioner can be applied to the first anchor assembly and the second anchor assembly to tension the anchor assemblies to a desired force or pressure. Following tensioning, the first suture 2316a and the second suture 2316b can be trimmed, such as near the first anchor sleeve 2314a and the second anchor sleeve 2314b, respectively.

Such a procedure can provide a minimally invasive process for repairing a fractured rib while placing an extrathoracic plate without deflating or "dropping" a lung for access.

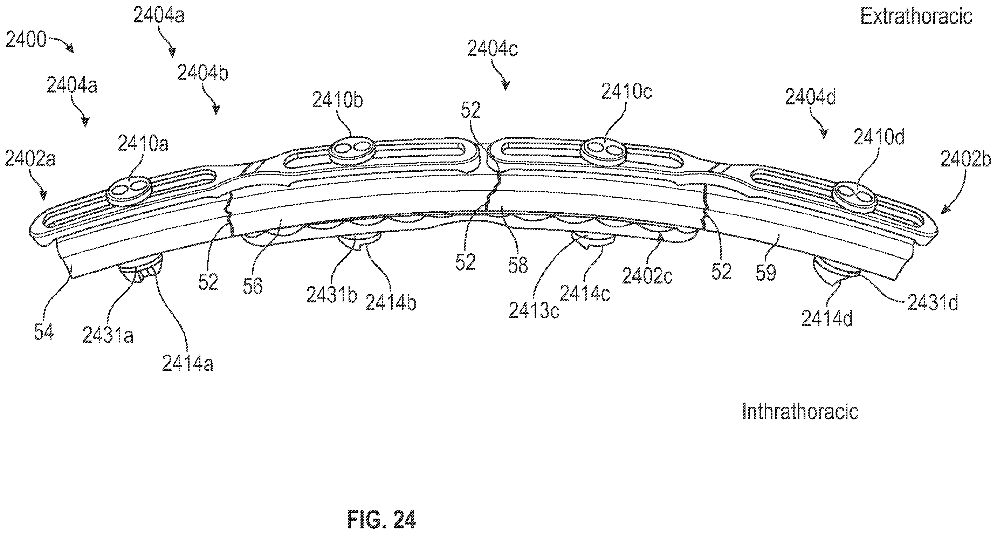

FIG. 24 illustrates a perspective view of an implant assembly 2400 installed in a rib 50 using a hybrid intrathoracic or intrathoracic approach. FIG. 24 also shows that the rib 50 includes fractures 52. The rib can include a first rib portion 54, a second rib portion 56, a third rib portion 58, and a fourth rib portion 59. FIG. 24 also shows orientation indicators Extrathoracic and Intrathoracic. FIG. 24 shows how the devices and methods discussed herein can be used to secure plates to opposing sides of the rib 50, such as in a stacking or railroading method.

More specifically, FIG. 24 shows how a first plate 2402a can be secured to an extrathoracic side of the first rib portion 54 and the rib portion 56 spanning a first fracture 52, a second plate 2402b can be secured to an extrathoracic side of the third portion 58 and the fourth rib portion 59 spanning a second fracture 52, and a third plate 2402c can be secured to an intrathoracic side of the rib portion 56 and the third portion 58 spanning a third fracture 52. In this way, multiple plates can be used to repair a multi-segment or multi-fracture rib using a relatively small number of holes.

For example, an anchor assembly 2404a (including a bracket 2410a, an anchor sleeve 2414a, and a washer 2431a) can be secured to the first plate 2402a and the first rib portion 54 through a bore of the first rib portion 54. An anchor assembly 2404b (including a bracket 2410b, an anchor sleeve 2414b, and a washer 2431b) can be secured to the rib portion 56, the first plate 2402a, and the third plate 2402c through a bore of the rib portion 56. An anchor assembly 2404c (including a bracket 2410c, an anchor sleeve 2414c, and a washer 2431c) can be secured to the rib portion 58, the second plate 2402b, and the third plate 2402c through a bore of the rib portion 58. And, an anchor assembly 2404d (including a bracket 2410d, an anchor sleeve 2414d, and a washer 2431d) can be secured to the third plate 2402c and the fourth rib portion 59 through a bore of the fourth rib portion 59. In this way, three plates or implants can be secured to the four rib segments using only four anchor assembly and optionally without screws.

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. In the following claims, the terms "first," "second," and "third," etc. are used merely as labels, and are not intended to impose numerical requirements on their objects.

The above description is intended to be illustrative, and not restrictive. The scope of the invention should be determined with reference to the appended claims.

## Claims

1. An implant assembly (100) securable to a human rib, the implant assembly comprising:
an implant including an elongate body (107) engageable with a surface of the rib, the elongate body defining a first bore (108a) and a second bore (108b) each extending at least partially through the body;
an anchor (112) insertable at least partially into a hole formed in the rib and at least partially into the first bore; and
a bracket (110) engageable with the implant and configured to receive at least a portion of the anchor therethrough, the anchor operable to secure the bracket and the anchor to the implant and the rib;
**characterized in that** the anchor comprises:
an anchor sleeve (114) defining a longitudinal bore extending at least partially therethrough, the anchor sleeve deformable from a first shape to a second shape; and
a suture (116) extending at least partly through the longitudinal bore to couple the sleeve to the suture, the suture including a first free end (830) and a second free end (832) operable to deform the anchor sleeve to the second shape;
wherein the suture comprises:
a first portion (836) including the first free end and a second portion (838) including the second free end, the first free end and the second free end connected to each other and operable to deform the anchor sleeve to the second shape;, wherein the suture defines a lumen (844) extending therethrough, the suture including a third portion (840) defining a first end bore (846) and a first side bore (848), the suture including a fourth portion (842) defining a second end bore (850) and a second side bore (852), the first portion extending into the first end bore and into the third portion and the lumen, the first portion transitioning into the fourth portion, the first portion, the third portion, and the fourth portion together forming a first loop, the fourth portion transitioning into the third portion and the third portion transitioning into the second portion, the fourth portion, the third portion, and the second portion together forming a second loop, the second portion extending through the second side bore and into the lumen and the fourth portion, the second portion extending through the second end bore and out of the lumen to connect to the first portion, and the first free end and the second free end operable to adjust a size of the first loop and a size of the second loop.

2. The implant assembly of claim 1, wherein the first free end and the second free end extend through the bracket and are operable to deform the anchor sleeve and adjust the size of the first loop and the size of the second loop.

3. The implant assembly of claim 1 or 2, wherein the bracket defines a bracket bore extending at least partially therethrough, and wherein the suture extends at least partially through the bracket bore to secure the anchor to the bracket.

4. The implant assembly of any of claims 1-3, wherein the first free end and the second free end extend through the anchor sleeve and are operable to deform the anchor sleeve and adjust the size of the first loop and the size of the second loop.

5. The implant assembly of claim 4, wherein the anchor comprises:
a leader (318) connected to the anchor sleeve and insertable through the hole of the rib and the bore of the implant to draw the anchor sleeve through the hole of the rib and the bore of the implant.

6. The implant assembly of claim 5, wherein the anchor comprises:
a tag (320) connected to the leader and operable to release the leader from the anchor sleeve.

7. The implant assembly of any of claims 1-6, wherein the first bore of the implant defines a longitudinal slot extending along the elongate body of the implant, at least one of the anchor and the bracket movable along the longitudinal slot to move the anchor and the bracket with respect to the implant.

8. The implant of claim 7, wherein the bracket includes a head and a projection, the head engageable with a surface of the implant, and the projection at least partially insertable into the slot to limit rotation of the bracket with respect to the implant.

9. The implant of claim 8, wherein the second bore of the implant defines a second longitudinal slot extending along the elongate body of the implant.

10. The implant of claim 9, wherein the projection comprises:
a barrel (1526) threadably securable to the bracket, the barrel insertable at least partially into the longitudinal slot, the barrel defining a barrel bore configured to receive at least a portion of the anchor therethrough.

11. The implant of claim 10, wherein the barrel includes a projection extending at least partially into the barrel bore, the projection configured to engage the anchor to allow movement of the anchor with respect to the barrel in a first direction and configured to limit movement of the anchor with respect to the barrel in a second direction opposite the first direction.

## Patentansprüche

1. Implantatanordnung (100), die an einer menschlichen Rippe befestigbar ist, wobei die Implantatanordnung Folgendes umfasst:
ein Implantat, das einen länglichen Körper (107) enthält, der mit einer Oberfläche der Rippe in Eingriff bringbar ist, wobei der längliche Körper eine erste Bohrung (108a) und eine zweite Bohrung (108b) definiert, die sich jeweils mindestens teilweise durch den Körper erstrecken;
eine Verankerung (112), die mindestens teilweise in ein in der Rippe ausgebildetes Loch und mindestens teilweise in die erste Bohrung einsetzbar ist; und
eine Halterung (110), die mit dem Implantat in Eingriff bringbar und dazu konfiguriert ist, mindestens einen Abschnitt der Verankerung dadurch aufzunehmen, wobei die Verankerung betätigbar ist, um die Halterung und die Verankerung an dem Implantat und der Rippe zu befestigen;
**dadurch gekennzeichnet, dass** die Verankerung Folgendes umfasst:
eine Verankerungshülse (114), die eine Längsbohrung definiert, die sich mindestens teilweise dadurch erstreckt, wobei die Verankerungshülse von einer ersten Gestalt in eine zweite Gestalt verformbar ist; und
einen Faden (116), der sich mindestens teilweise durch die Längsbohrung erstreckt, um die Hülse an den Faden zu koppeln, wobei der Faden ein erstes freies Ende (830) und ein zweites freies Ende (832) enthält, die betätigbar sind, um die Verankerungshülse in die zweite Gestalt zu verformen;
wobei der Faden Folgendes umfasst:
einen ersten Abschnitt (836), der das erste freie Ende enthält, und einen zweiten Abschnitt (838), der das zweite freie Ende enthält, wobei das erste freie Ende und das zweite freie Ende miteinander verbunden sind und betätigbar sind, um die Verankerungshülse in die zweite Gestalt zu verformen, wobei der Faden ein Lumen (844) definiert, das sich dadurch erstreckt, wobei der Faden einen dritten Abschnitt (840) enthält, der eine erste Endbohrung (846) und eine erste Seitenbohrung (848) definiert, wobei der Faden einen vierten Abschnitt (842) enthält, der eine zweite Endbohrung (850) und eine zweite Seitenbohrung (852) definiert, der erste Abschnitt sich in die erste Endbohrung und in den dritten Abschnitt und das Lumen erstreckt, wobei der erste Abschnitt in den vierten Abschnitt übergeht, der erste Abschnitt, der dritte Abschnitt und der vierte Abschnitt zusammen eine erste Schlaufe ausbilden, der vierte Abschnitt in den dritten Abschnitt übergeht und der dritte Abschnitt in den zweiten Abschnitt übergeht, wobei der vierte Abschnitt, der dritte Abschnitt und der zweite Abschnitt zusammen eine zweite Schlaufe ausbilden, der zweite Abschnitt sich durch die zweite Seitenbohrung und in das Lumen und den vierten Abschnitt erstreckt, wobei sich der zweite Abschnitt durch die zweite Endbohrung und aus dem Lumen erstreckt, um sich mit dem ersten Abschnitt zu verbinden, und wobei das erste freie Ende und das zweite freie Ende betätigbar sind, um eine Größe der ersten Schlaufe und eine Größe der zweiten Schlaufe einzustellen.

2. Implantatanordnung nach Anspruch 1, wobei sich das erste freie Ende und das zweite freie Ende durch die Halterung erstrecken und betätigbar sind, um die Verankerungshülse zu verformen und die Größe der ersten Schlaufe und die Größe der zweiten Schlaufe einzustellen.

3. Implantatanordnung nach Anspruch 1 oder 2, wobei die Halterung eine Halterungsbohrung definiert, die sich mindestens teilweise dadurch erstreckt, und wobei sich der Faden mindestens teilweise durch die Halterungsbohrung erstreckt, um die Verankerung an der Halterung zu befestigen.

4. Implantatanordnung nach einem der Ansprüche 1-3, wobei sich das erste freie Ende und das zweite freie Ende durch die Verankerungshülse erstrecken und betätigbar sind, um die Verankerungshülse zu verformen und die Größe der ersten Schlaufe und die Größe der zweiten Schlaufe einzustellen.

5. Implantatanordnung nach Anspruch 4, wobei die Verankerung Folgendes umfasst:
eine Leitschnur (318), die mit der Verankerungshülse verbunden ist und durch das Loch der Rippe und die Bohrung des Implantats einsetzbar ist, um die Verankerungshülse durch das Loch der Rippe und die Bohrung des Implantats zu ziehen.

6. Implantatanordnung nach Anspruch 5, wobei die Verankerung Folgendes umfasst:
eine Schlinge (320), die mit der Leitschnur verbunden ist und betätigbar ist, um die Leitschnur von der Verankerungshülse zu lösen.

7. Implantatanordnung nach einem der Ansprüche 1-6, wobei die erste Bohrung des Implantats einen Längsschlitz definiert, der sich entlang des länglichen Körpers des Implantats erstreckt, wobei mindestens eine von der Verankerung und der Halterung entlang des Längsschlitzes bewegbar ist, um die Verankerung und die Halterung in Bezug auf das Implantat zu bewegen.

8. Implantat nach Anspruch 7, wobei die Halterung einen Kopf und einen Vorsprung enthält, wobei der Kopf mit einer Oberfläche des Implantats in Eingriff bringbar ist und der Vorsprung mindestens teilweise in den Schlitz einsetzbar ist, um eine Drehung der Halterung in Bezug auf das Implantat zu begrenzen.

9. Implantat nach Anspruch 8, wobei die zweite Bohrung des Implantats einen zweiten Längsschlitz definiert, der sich entlang des länglichen Körpers des Implantats erstreckt.

10. Implantat nach Anspruch 9, wobei der Vorsprung Folgendes umfasst:
einen Zylinder (1526), der schraubbar an der Halterung befestigbar ist, wobei der Zylinder mindestens teilweise in den Längsschlitz einsetzbar ist, wobei der Zylinder eine Zylinderbohrung definiert, die dazu konfiguriert ist, mindestens einen Abschnitt der Verankerung dadurch aufzunehmen.

11. Implantat nach Anspruch 10, wobei der Zylinder einen Vorsprung enthält, der sich mindestens teilweise in die Zylinderbohrung erstreckt, wobei der Vorsprung dazu konfiguriert ist, die Verankerung in Eingriff zu nehmen, um eine Bewegung der Verankerung in Bezug auf den Zylinder in einer ersten Richtung zuzulassen, und dazu konfiguriert ist, eine Bewegung der Verankerung in Bezug auf den Zylinder in einer zweiten Richtung, die der ersten Richtung entgegengesetzt ist, zu begrenzen.

## Revendications

1. Ensemble implant (100) pouvant être fixé à une côte humaine, l'ensemble implant comprenant :
un implant comprenant un corps allongé (107) pouvant se mettre en prise avec une surface de la nervure, le corps allongé définissant un premier alésage (108a) et un second alésage (108b) s'étendant chacun au moins partiellement à travers le corps ;
un élément d'ancrage (112) pouvant être inséré au moins partiellement dans un trou formé dans la nervure et au moins partiellement dans le premier alésage ; et
un support (110) pouvant être mis en prise avec l'implant et conçu pour recevoir au moins une partie de l'élément d'ancrage en son travers, l'élément d'ancrage servant à fixer le support et l'élément d'ancrage à l'implant et à la nervure ;
**caractérisé en ce que** l'élément d'ancrage comprend :
un manchon d'élément d'ancrage (114) définissant un alésage longitudinal s'étendant au moins partiellement en son travers, le manchon d'élément d'ancrage pouvant être déformé d'une première forme à une seconde forme ; et
une suture (116) s'étendant au moins partiellement à travers l'alésage longitudinal de manière à coupler le manchon à la suture, la suture comprenant une première extrémité libre (830) et une seconde extrémité libre (832) servant à déformer le manchon d'élément d'ancrage en la seconde forme ;
ladite suture comprenant :
une première partie (836) comprenant la première extrémité libre et une deuxième partie (838) comprenant la seconde extrémité libre, la première extrémité libre et la seconde extrémité libre étant reliées l'une à l'autre et servant à déformer le manchon d'élément d'ancrage en la seconde forme ; ladite suture définissant une lumière (844) s'étendant en son travers, ladite suture comprenant une troisième partie (840) définissant un premier alésage d'extrémité (846) et un premier alésage latéral (848), ladite suture comprenant une quatrième partie (842) définissant un second alésage d'extrémité (850) et un second alésage latéral (852), la première partie s'étendant dans le premier alésage d'extrémité et dans la troisième partie et la lumière, la première partie passant dans la quatrième partie, la première partie, la troisième partie et la quatrième partie formant ensemble une première boucle, la quatrième partie passant dans la troisième partie et la troisième partie passant dans la deuxième partie, la quatrième partie, la troisième partie et la deuxième partie formant ensemble une seconde boucle, la deuxième partie s'étendant à travers le second alésage latéral et dans la lumière et la quatrième partie, la deuxième partie s'étendant à travers le second alésage d'extrémité et hors de la lumière pour se raccorder à la première partie, et la première extrémité libre et la seconde extrémité libre servant à ajuster la taille de la première boucle et la taille de la seconde boucle.

2. Ensemble implant selon la revendication 1, ladite première extrémité libre et ladite seconde extrémité libre s'étendant à travers le support et servant à déformer le manchon d'élément d'ancrage et à ajuster la taille de la première boucle et la taille de la seconde boucle.

3. Ensemble implant selon la revendication 1 ou 2, ledit support définissant un alésage de support s'étendant au moins partiellement en son travers, et ladite suture s'étendant au moins partiellement à travers l'alésage de support de manière à fixer l'élément d'ancrage au support.

4. Ensemble implant selon l'une quelconque des revendications 1 à 3, ladite première extrémité libre et ladite seconde extrémité libre s'étendant à travers le manchon d'élément d'ancrage et servant à déformer le manchon d'élément d'ancrage et à ajuster la taille de la première boucle et la taille de la seconde boucle.

5. Ensemble implant selon la revendication 4, ledit élément d'ancrage comprenant :
une amorce (318) reliée au manchon d'élément d'ancrage et pouvant être inséré à travers le trou de la nervure et l'alésage de l'implant de manière à tirer le manchon d'élément d'ancrage à travers le trou de la nervure et l'alésage de l'implant.

6. Ensemble implant selon la revendication 5, ledit élément d'ancrage comprenant :
une étiquette (320) reliée à l'amorce et servant à libérer l'amorce du manchon d'élément d'ancrage.

7. Ensemble implant selon l'une quelconque des revendications 1 à 6, ledit premier alésage de l'implant définissant une fente longitudinale s'étendant le long du corps allongé de l'implant, au moins un élément parmi l'élément d'ancrage et le support se déplaçant le long de la fente longitudinale de manière à déplacer l'élément d'ancrage et le support par rapport à l'implant.

8. Implant selon la revendication 7, ledit support comprenant une tête et une saillie, la tête pouvant se mettre en prise avec une surface de l'implant, et la saillie pouvant être au moins partiellement insérée dans la fente de manière à limiter la rotation du support par rapport à l'implant.

9. Implant selon la revendication 8, ledit second alésage de l'implant définissant une seconde fente longitudinale s'étendant le long du corps allongé de l'implant.

10. Implant selon la revendication 9, ladite saillie comprenant :
un cylindre (1526) pouvant être fixé par vissage au support, le cylindre pouvant être inséré au moins partiellement dans la fente longitudinale, le cylindre définissant un alésage de cylindre conçu pour recevoir au moins une partie de l'élément d'ancrage en son travers.

11. Implant selon la revendication 10, ledit cylindre comprenant une saillie s'étendant au moins partiellement dans l'alésage du cylindre, la saillie étant conçue pour se mettre en prise avec l'élément d'ancrage de manière à permettre le mouvement de l'élément d'ancrage par rapport au cylindre suivant une première direction et étant conçue pour limiter le mouvement de l'élément d'ancrage par rapport au cylindre suivant une seconde direction opposée à la première direction.
